# EUROPEAN PATENT APPLICATION

(11) **EP 4 768 507 A1**
(43) Date of publication of application: **01.07.2026**
(21) Application number: 24861984.3
(22) Date of filing: 04.09.2024
(51) Int. Cl.: C07K 16/46, C12N 15/13, A61K 39/395, A61P 35/00, G01N 33/68

(54) **BISPECIFIC ANTIBODY AND USE THEREOF**

(30) Priority: 06.09.2023 CN 202311146466
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Potter Clarkson
(86) International application number: PCT/CN2024/116778
(87) International publication number: WO 2025/051141

(57) **Abstract**

Provided is a bispecific antibody targeting CD3 and a tumor target and a use of the bispecific antibody. The bispecific antibody comprises a first antigen binding region, a second antigen binding region, and an Fc portion; the Fc portion comprises a first Fc peptide fragment and a second Fc peptide fragment; the first antigen binding region comprises an scFv fragment and a binding protein or fragment thereof of a first molecule, the binding protein or fragment thereof of the first molecule is linked to the N-terminal of the scFv fragment, and the C-terminal of the scFv fragment is linked to the N-terminal of the first Fc peptide fragment; the second antigen binding region comprises a binding protein or fragment thereof of a second molecule, and the binding protein or the fragment thereof of the second molecule is linked to the N-terminal of the second Fc peptide fragment fragment; and the first molecule and the second molecule are the identical, and the scFv fragment has CD3 binding activity. The bispecific antibody has weak binding to T-cells, strong binding to tumor cells, good safety, high anticancer activity, and significant value for clinical use and drug development.

## Description

### FIELD

The present disclosure belongs to the field of biomedicine. Particularly, the present disclosure relates to a bispecific antibody and use thereof, and more particularly, to an antibody, a nucleic acid molecule, a vector or transformant, a cell, a pharmaceutical composition, and a kit, and use thereof.

### BACKGROUND

Cancer is a major disease that affects human survival and development. According to the latest data, there are about 19 million new cancer cases and around 10 million cancer-related deaths worldwide each year, with both incidence and mortality rates showing an upward trend. In addition to surgical resection, traditional cancer treatment methods such as chemotherapy and radiotherapy have serious side effects and are prone to recurrence. In recent years, immunotherapy, including tumor-targeting antibodies, immune checkpoint antibodies, and bispecific antibodies, etc., has become a new hotspot and and a new hope in an anti-cancer aspect. The immunotherapy, represented by PD-1/L1, has shown great potential. However, even the PD-1/L1 therapy with broadest approved indications currently has an overall response rate of only 30%, and many more patients cannot benefit from it. One of the main reasons is that immune checkpoint therapies are ineffective against "cold tumors". T cells recognize neoantigens, namely, antigens resulting from tumor gene mutations, through T cell receptors (TCRs) on their surface. However, some tumors exhibit a low gene mutation frequency and limited neoantigen diversity, which are referred to as the "cold tumors". Current immune checkpoint therapies, such as the PD-1/L1 therapy, can achieve anti-cancer effects by restoring intrinsic function of T cells. However, in the "cold tumors", T cells cannot effectively recognize the tumors, making the immune checkpoint therapies ineffective against the "cold tumors".

CD3-based bispecific antibodies (hereinafter referred to as "CD3 bispecific antibodies") can promote T cell activation and kill tumors by recruiting T cells to reach the tumor site, and bridging T cells with tumors. Such bispecific antibodies do not require neoantigens and can guide T cells to kill the "cold tumors". The CD3 bispecific antibodies can trigger strong activation signals upon binding to T cells and tumor cells, and they can thus also "ignore" inhibitory signals of immune checkpoint molecules to some extent. However, the CD3 bispecific antibodies can also promote generation of a large number of pro-inflammatory cytokines, such as TNFα and IL-6, triggering a strong cytokine storm and excessive immune response, which cause damage to the body and even potential life-threatening in severe cases. Therefore, the CD3-based bispecific antibodies have promising clinical applications, but their safety needs to be further improved.

One approach to solve the safety issues of the CD3 bispecific antibodies is to increase their affinity for binding to tumor targets while reducing their affinity for CD3 and weakening their binding to T cells simultaneously, thereby enabling the CD3 bispecific antibody drugs to distribute more extensively to the tumor site, increasing local drug concentrations at the tumor site, reducing a peripheral drug concentration, and lowering off-target toxicity. Therefore, there is an urgent need in clinical practice to develop CD3 bispecific antibodies with better safety and higher clinical application value.

### SUMMARY

The present disclosure aims to solve at least one of the technical problems existing in the related art. To this end, one object of the present disclosure is to provide a bispecific antibody targeting CD3 and a tumor target. The bispecific antibody can bind to both CD3 and the tumor target and has low CD3 binding activity, weak binding to T cells, and a weak capability to bridge T cells and tumor cells. At the same time, it can effectively promote an activity of peripheral blood mononuclear cells (PBMCs) for killing tumors, has enhanced T cell activation performance, and can promote drug distribution to a tumor site rather than to periphery or lymphoid tissues. Thus, the bispecific antibody of the present disclosure has good safety, high anti-cancer activity, and high value in clinical application and drug development.

The present disclosure is based on the inventors' discoveries and understanding of the following facts and problems.

In order to improve the safety of CD3 and major histocompatibility complex class I chain related polypeptide A (MICA) bispecific antibodies, the inventors have obtained a CD3 and MICA bispecific antibody with a new configuration through extensive screening and experimental validation. Further experimental results demonstrate that the CD3 and MICA bispecific antibody has weak binding to T cells and strong binding to tumor cells as well as high pro-killing activity, effectively promoting PBMCs for killing tumors and exhibiting good anti-cancer activity. Moreover, the bispecific antibody has reduced T cell binding activity, high T cell activation performance, higher safety, and satisfactory value in clinical application and drug development.

Therefore, in a first aspect, the present disclosure provides a bispecific antibody. According to embodiments of the present disclosure, the bispecific antibody includes a first antigen-binding region, including an scFv fragment and a binding protein or fragment thereof of a first molecule; a second antigen-binding region, including a binding protein or fragment thereof of a second molecule; and an Fc portion, including a first Fc peptide fragment and a second Fc peptide fragment, wherein: the binding protein or fragment thereof of the first molecule is linked to an N-terminus of the scFv fragment, and a C-terminus of the scFv fragment is linked to an N-terminus of the first Fc peptide fragment; the binding protein or fragment thereof of the second molecule is linked to an N-terminus of the second Fc peptide fragment; and the first molecule and the second molecule are identical, and the scFv fragment has CD3 binding activity.

The bispecific antibody according to the embodiments of the present disclosure can bind to CD3 and a tumor target and has low CD3 binding activity, weak binding to T cells, and a weak capability to bridge T cells and tumor cells. At the same time, it can effectively promote an activity of peripheral blood mononuclear cells (PBMCs) for killing tumors, has enhanced T cell activation performance, and can promote drug distribution to a tumor site rather than to periphery or lymphoid tissues. Thus, the bispecific antibody of the present disclosure has good safety, high anti-cancer activity, and high value in clinical application and drug development.

In a second aspect, the present disclosure provides a nucleic acid molecule. According to embodiments of the present disclosure, the nucleic acid molecule encodes the aforementioned bispecific antibody. The nucleic acid according to the embodiments of the present disclosure can encode a bispecific antibody that can simultaneously target CD3 and a tumor target.

In a third aspect, the present disclosure provides a vector or transformant. According to embodiments of the present disclosure, the vector or transformant includes the aforementioned nucleic acid. Therefore, the aforementioned bispecific antibody can be effectively expressed using the constructed vector or transformant.

In a fourth aspect, the present disclosure provides a cell. According to embodiments of the present disclosure, the cell carries the aforementioned nucleic acid or the aforementioned vector or transformant, or expresses the aforementioned bispecific antibody. The cell according to the embodiments of the present disclosure is obtained by transfecting or transforming the vector or transformant, and can thus efficiently express the aforementioned bispecific antibody under a suitable condition.

In a fifth aspect, the present disclosure provides a pharmaceutical composition. According to embodiments of the present disclosure, the pharmaceutical composition includes the aforementioned bispecific antibody, the aforementioned nucleic acid, the aforementioned vector or transformant, or the aforementioned cell. According to the embodiments of the present disclosure, the bispecific antibody can effectively promote PBMC-mediated killing of tumors and has high anti-cancer activity, low CD3 binding activity, and good safety. The resulting medicament can be further used to prevent and/or treat a CD3-mediated disease and/or tumor target-mediated disease.

In a sixth aspect, the present disclosure provides a kit. According to embodiments of the present disclosure, the kit includes the aforementioned bispecific antibody, the aforementioned nucleic acid, the aforementioned vector or transformant, or the aforementioned cell. According to the embodiments of the present disclosure, the kit can bind to a CD3 protein and/or tumor target, and can thus effectively identify the CD3 protein and/or tumor target.

Those skilled in the art will understand that the features and advantages described above for the antibody, the nucleic acid, the vector or transformant, the cell, or the pharmaceutical composition are also applicable to the kit, and details thereof are omitted herein.

In a seventh aspect of the present disclosure, the present disclosure provides use of the aforementioned bispecific antibody, the aforementioned nucleic acid, the aforementioned vector or transformant, or the aforementioned cell in preparation of a kit for detecting CD3 and/or a tumor target.

Those skilled in the art will understand that the features and advantages described above for the antibody, the nucleic acid, the vector or transformant, the cell, or the pharmaceutical composition are also applicable to the use in the preparation of the kit, and details thereof are omitted herein.

In an eighth aspect of the present disclosure, the present disclosure provides use of the aforementioned bispecific antibody, the aforementioned nucleic acid molecule, the aforementioned carrier or transformant, the aforementioned cell, or the aforementioned pharmaceutical composition in preparation of a medicament for preventing and/or treating a CD3-mediated disease and/or tumor target-mediated disease.

Those skilled in the art will understand that the features and advantages described above for the antibody, the nucleic acid molecule, the vector or transformant, the cell, and the pharmaceutical composition are also applicable to the use, and details thereof are omitted herein.

Additional aspects and advantages of the present disclosure will be provided at least in part in the following description, or will become apparent in part from the following description, or can be learned from the practice of the present disclosure.

### BRIEF DESCRIPTION OF THE DRAWINGS

The foregoing and/or additional aspects and advantages of the present disclosure will become apparent and readily appreciated from the following description of the examples in conjunction with the accompanying drawings, in which:
FIG. 1 is a schematic diagram of a CD3×MICA bispecific antibody according to a specific embodiment of the present disclosure.
FIG. 2 is a graph showing the enzyme-linked immunosorbent assay (ELISA) results of binding of Cross3×h5A1002 1:1, Cross3×h5A1002 2:1, and UCHT1×h5A1002 2:1 antibodies to MICA protein according to a specific embodiment of the present disclosure.
FIG. 3 is a graph showing the ELISA results of binding of Cross3×h5A1002 1:1, Cross3×h5A1002 2:1, and UCHT1×h5A1002 2:1 antibodies to CD3E&D protein according to a specific embodiment of the present disclosure.
FIG. 4 is a graph showing the ELISA results of bridging of t CD3E&D protein and MICA protein by Cross3×h5A1002 1:1, Cross3×h5A1002 2:1, and UCHT1×h5A1002 2:1 antibodies according to a specific embodiment of the present disclosure.
FIG. 5 is a graph showing the flow cytometry results of binding of Cross3×h5A1002 1:1, Cross3×h5A1002 2:1, UCHT1×h5A1002 1:1, and UCHT1×h5A1002 2:1 antibodies to CD8+ T cells according to a specific embodiment of the present disclosure.
FIG. 6 is a graph showing the flow cytometry results of binding of Cross3 ×h5A1002 1:1, Cross3×h5A1002 2:1, UCHT1×h5A1002 1:1, and UCHT1×h5A1002 2:1 antibodies to HCT-15 colorectal cancer cells according to a specific embodiment of the present disclosure.
FIG. 7 is a graph showing the results of Cross3×h5A1002 1:1, Cross3×h5A1002 2:1, UCHT1×h5A1002 1:1, and UCHT1×h5A1002 2:1 antibodies promoting PBMC-mediated killing of HCT-15 colorectal cancer cells according to a specific embodiment of the present disclosure.
FIG. 8 is a graph showing the results of Cross3×h5A1002 1:1, Cross3×h5A1002 2:1, UCHT1×h5A1002 1:1, and UCHT1×h5A1002 2:1 antibodies promoting PBMC-mediated killing of NCI-H1299 lung cancer cells according to a specific embodiment of the present disclosure.
FIG. 9 is a graph showing the results of Cross3×h5A1002 1:1, Cross3×h5A1002 2:1, UCHT1×h5A1002 1:1, and UCHT1×h5A1002 2:1 antibodies promoting PBMC-mediated killing of PANC-1 pancreatic cancer cells according to a specific embodiment of the present disclosure.
FIG. 10 is a graph showing the results of Cross3×h5A1002 1:1, Cross3×h5A1002 2:1, UCHT1×h5A1002 1:1, and UCHT1×h5A1002 2:1 antibodies promoting PBMC-mediated killing of A498 renal cancer cells according to a specific embodiment of the present disclosure.
FIG. 11 is a graph showing the results of Cross3×h5A1002 1:1, Cross3×h5A1002 2:1, UCHT1×h5A1002 1:1, and UCHT1×h5A1002 2:1 antibodies promoting PBMC-mediated killing of PC-3 prostate cancer cells according to a specific embodiment of the present disclosure.
FIG. 12 is a graph showing the results of Cross3×h5A1002 1:1 and Cross3×h5A1002 2:1 antibodies promoting the secretion of immune-activating cytokines IL-2 and IFN-γ after co-incubation of PBMCs and HCT-15 colorectal cancer cells according to a specific embodiment of the present disclosure.
FIG. 13 is a graph showing the results of Cross3×h5A1002 2:1, UCHT1×h5A1002 2:1, and SP34×h5A1002 2:1 antibodies promoting PBMC-mediated killing of HCT-15 colorectal cancer cells according to a specific embodiment of the present disclosure.
FIG. 14 is a graph showing the results of Cross3×h5A1002 2:1, Cross3×1D5 1:1, and Cross3×1D5 2:1 antibodies promoting PBMC-mediated killing of HCT-15 colorectal cancer cells according to a specific embodiment of the present disclosure.
FIG. 15 is a graph showing the results of Cross3×h5A1002 2:1, Cross3×13A9 1:1, and Cross3×13A9 2:1 antibodies promoting PBMC-mediated killing of HCT-15 colorectal cancer cells according to a specific embodiment of the present disclosure.
FIG. 16 is a graph showing the results of Cross3×h5A1002 2:1, Cross3×619 1:1, and Cross3×619 2:1 antibodies promoting PBMC-mediated killing of HCT-15 colorectal cancer cells according to a specific embodiment of the present disclosure.
FIG. 17 is a graph showing the results of in vivo efficacy of a Cross3×h5A1002 2:1 antibody in an immune-reconstituted mouse A-375 human melanoma model according to a specific embodiment of the present disclosure.
FIG. 18 a graph showing the results of in vivo efficacy of a Cross3×h5A1002 2:1 antibody in an immune-reconstituted mouse NCI-H1299 human lung cancer model according to a specific embodiment of the present disclosure.
FIG. 19 a graph showing the results of in vivo efficacy of a Cross3×h5A1002 2:1 antibody in an immune-reconstituted mouse PC-3 human prostate cancer model according to a specific embodiment of the present disclosure.

### DETAILED DESCRIPTION OF THE EMBODIMENTS

Embodiments of the present disclosure will now be described in detail, examples of which are illustrated in the accompanying drawings. The embodiments described below with reference to the figures are exemplary and are intended to be illustrative of the present disclosure and are not to be construed as limiting the present disclosure.

Furthermore, the terms "first" and "second" are used for descriptive purposes only and are not to be construed as indicating or implying relative importance, or implicitly indicating the number of technical features indicated. Thus, a feature defined with "first" or "second" may explicitly or implicitly comprises at least one feature. In the description of the present disclosure, the meaning of "plurality/more" is at least two, e.g. two, three, etc. unless specifically limited otherwise.

The endpoints of the ranges and any values disclosed herein are not limited to the precise range or value, and such ranges or values should be understood to include values approximating such ranges or values. For numerical ranges, the endpoints of each range, the endpoints of each range and the individual point values, and the individual point values, can be combined with each other to obtain one or more new numerical ranges, and such numerical ranges are to be considered to be specifically disclosed herein.

### Terms and Definitions

For an easier understanding of the present disclosure, certain technical and scientific terms are specifically defined below. Unless obviously and clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

In the present disclosure, the term "antibody" generally refers to an antibody that can recognize one or more antigenic epitopes, including but not limited to monoclonal antibodies, polyclonal antibodies, dimers, multimers, bispecific antibodies, heavy chain-only antibodies, triple-chain antibodies, single-chain Fv (scFv), nanobodies, etc., and further includes antibody fragments, as long as they exhibit the desired biological activity. The antibody may be murine, human, humanized, chimeric, or from other species. The antibody may refer to full-length heavy chains, full-length light chains, intact immunoglobulin molecules, or immunologically active portions of any one of these polypeptides, i.e., molecules or portions thereof that include an antigen-binding site that immunologically specifically binds to a target antigen of interest. Such targets include, but are not limited to, cancer cells or cells that generate autoantibodies associated with autoimmune diseases.

In the present disclosure, in a variable region, certain regions have a higher degree of variation in amino acid composition and arrangement, referred to as a Hypervariable region (HVR), which is a location where an antigen and an antibody bind, and thus also referred to as complementarity-determining region (CDR). There are three CDR regions in both a heavy-chain variable region and a light-chain variable region. For example, these typically includes: amino acid residues near 23-34 (L1), 50-56 (L2), and 89-97 (L3) in the light chain variable region, and amino acid residues near 31-35B (H1), 50-65 (H2), and 95-102 (H3) in the heavy chain variable region; and/or amino acid residues from "hypervariable loops" (e.g., amino acid residues near 26-32 (L1), 50-52 (L2), and 91-96 (L3) in the light chain variable region, and amino acid residues near 26-32 (H1), 53-55 (H2), and 96-101 (H3) in the heavy chain variable region).

In the present disclosure, the term "anti-CD3 antibody" refers to an antibody that can bind to CD3, which is also referred to herein as a "CD3-binding antibody". The term "anti-MICA antibody" refers to an antibody that can bind to MICA, which is also referred to herein as a "MICA-binding antibody".

In the present disclosure, the term "antigen-binding fragment" is equivalent to "antibody fragment" or "antigen-binding antibody fragment", and it may include a portion of a complete antibody, typically an antigen-binding region or a variable region, including, but not limited to, Fv, scFv, Fab, Fab', Fab'-SH, single chain Fab fragment (scFab), F(ab')₂, scFv-Fc fragment, or bispecific antibodies (BsAbs), linear antibodies, or any fragment which should be capable of increasing the half-life by chemical modification, e.g. addition of polyalkylene glycols, such as polyethylene glycol ("PEGylation, PEG") (referred to as pegylated fragments of Fv-PEG, scFv-PEG, Fab-PEG, F(ab')₂-PEG or Fab'-PEG) ("PEG" is polyethylene glycol) or by incorporation into liposomes.

In the present disclosure, the term "chimeric antibody" refers to a recombinant antibody obtained by replacing the amino acid sequence of the constant region of a monoclonal antibody from one species (e.g. mouse) with the constant region of an antibody from another species (e.g. human) using recombinant DNA technology.

In the present disclosure, the term "humanized antibody" refers to a recombinant antibody obtained by completely replacing the amino acid sequences of the constant region and the non-CDR (Fv framework region (FR)) in the variable region of a monoclonal antibody from one species (e.g. mouse) with the amino acid sequences of the constant region and non-CDR in the variable region of an antibody from another species (e.g. human) using recombinant DNA technology. That is, when the constant region of an antibody is humanized, it is referred to as a chimeric antibody, and when all the amino acid sequences of the constant and the non-CDR in the variable region are, for example, humanized, it is referred to as a humanized antibody. Methods of humanization may be carried out with reference to conventional antibody engineering techniques and will not be described in detail herein.

For nucleotides, the terms "homology," "identity," or "similarity" are used to describe or compare the degree of nucleotide similarity between two or more nucleotide sequences. The percentage of "sequence homology" between a first sequence and a second sequence may be calculated by dividing the number of nucleotides in the first sequence that are identical to the nucleotides at the corresponding positions. [Nucleotides in the second sequence] minus [total number of nucleotides in the first sequence] and then multiplied by [100%], wherein the deletion, insertion, substitution, or addition of each nucleotide in the second nucleotide sequence relative to the first nucleotide sequence is considered to be a difference in a single nucleotide (position). Alternatively, the degree of sequence identity between two or more nucleotide sequences can be calculated using known computer algorithms for sequence alignment, such as NCBI Blast v2.0. Some other techniques, computer algorithms, and settings for determining the degree of sequence identity are described, for example, in WO 04/037999, EP 0 967 284, EP 1 085 089, WO 00/55318, WO 00/78972, WO 98/49185 and GB 2357768-A.

For peptides, the terms "(substantial) homology," "identity," or "similarity" are used to describe or compare the degree of amino acid similarity between two or more polypeptides or designated sequences thereof when optimally aligned and compared (with appropriate insertions or deletions of nucleotides). The % homology between two sequences varies with the number of identical positions shared by the sequences at optimal alignment (i.e.% homology = number of identical positions/total number of positions × 100), where optimal alignment is determined by taking into account the number of gaps that need to be introduced, and the length of each gap, to achieve optimal alignment of the two sequences. Sequence comparison and percent identity determination between two sequences can be accomplished using mathematical algorithms, as described in the non-limiting examples below.

In the present disclosure, a "an amino acid sequence in conservative modification form" refers to an amino acid modification that does not significantly affect or alter the binding characteristics of an antibody including the amino acid sequence, and the modification includes the substitutions, additions, and deletions of the amino acids. Modifications can be introduced into the antibodies of the present disclosure by standard techniques such as site-directed mutagenesis and PCR-mediated mutagenesis. Conservative amino acid substitution refers to the substitution in which the amino acid residues are replaced by amino acid residues with similar side chains. Families of amino acid residues having similar side chains have been identified in the art. These families include amino acids with basic side chains, e.g. lysine, arginine, histidine, amino acids with acidic side chains, e.g. aspartic acid, and glutamic acid, amino acids with uncharged polar side chains, e.g. glycine, asparagine, glutamine, serine, threonine, tyrosine, cysteine, and tryptophan, amino acids with nonpolar side chains, e.g. alanine, valine, leucine, isoleucine, proline, phenylalanine, and methionine, amino acids with β-branched side chains, e.g. threonine, valine, isoleucine, and amino acids with aromatic side chains, e.g. tyrosine, phenylalanine, tryptophan, and histidine.

In the present disclosure, the term "vector" generally refers to a nucleic acid molecule capable of being inserted into a suitable host for self-replication, which transfers the inserted nucleic acid molecule into and/or between host cells. The vector may include vectors primarily used for inserting DNA or RNA into cells, vectors primarily used for replicating DNA or RNA, and vectors primarily used for expressing DNA or RNA through transcription and/or translation. The vector also includes vectors that have multiple functions as described above. The vector may be a polynucleotide capable of being transcribed and translated into a polypeptide when introduced into a suitable host cell. Typically, the vector can generate a desired expression product by culturing a suitable host cell including the vector.

In the present disclosure, the term "pharmaceutical composition" generally refers to a unit dose form and can be prepared by any one of the methods well known in the pharmaceutical field. All such methods include the step of combining the active ingredient with the carrier which constitutes one or more accessory ingredients. Typically, the composition is prepared by uniformly and thoroughly combining an active bispecific antibody with a liquid carrier, a finely divided solid carrier, or a combination thereof.

In the present disclosure, the term "pharmaceutically acceptable ingredient" refers to substance that is suitable for use in humans and/or mammals without undue adverse side effects (such as toxicity, irritation, and allergic response), i.e. one that has a reasonable benefit/risk ratio.

In the present disclosure, the term "pharmaceutical ally acceptable adjuvant" may include any solvent, solid excipient, diluent, or other liquid excipients etc., suitable for specific target dosage forms. Except for any cases where conventional adjuvants are incompatible with the bispecific antibody of the present disclosure, such as any adverse biological effects that may arise or harmful interactions with any other components of the pharmaceutically acceptable composition, the use of such adjuvants is also within the scope of the present disclosure.

In the present disclosure, the term "administering/administration" refers to introduction of a predetermined amount of a substance into a patient via a suitable mode. The antibody or antigen-binding fragment, recombinant protein, bispecific antibody, conjugate, or pharmaceutical composition of the present disclosure can be administered via any common route, as long as it can reach intended tissues. Various administration modes are contemplated, including intraperitoneal, intravenous, intramuscular, and subcutaneous injection, but the present disclosure is not limited to these exemplified administration modes. Preferably, the composition of the present disclosure is administered via intravenous or subcutaneous injection.

In the present disclosure, the term "treat/treatment" refers to treatment intended to achieve a desired pharmacological and/or physiological effect. Said effect may be preventive in terms of completely or partially prevention of a disease or its symptoms, and/or therapeutic in terms of partially or completely cure of a disease and/or adverse effects caused by the disease. The term "treat/treatment" as used herein encompasses diseases in mammals, particularly humans, including: (a) preventing an onset of a disease or disorder in individuals who are susceptible to the disease but have not yet been diagnosed with it; (b) suppressing the disease, for example, halting its progression; or (c) alleviating the disease, for example, reducing symptoms associated with the disease. The term "treat/treatment" as used herein encompasses any administration of a medicament or a bispecific antibody to an individual for treating, curing, alleviating, ameliorating, relieving, or inhibiting the individual's disease, including but not limited to administering a pharmaceutical composition including the bispecific antibody described herein to an individual in need thereof.

As used herein, the term "effective amount" or "effective dose" refers to an amount that may produce functions or activity to humans and/or animals and is acceptable to the humans and/or animals.

The present disclosure provides a bispecific antibody targeting CD3 and a tumor target, a nucleic acid molecule, a vector or transformant, a cell, a pharmaceutical composition, a kit, and use thereof, and details thereof will be described below.

### Bispecific Antibody

The present disclosure provides a bispecific antibody. The bispecific antibody includes a first antigen-binding region, a second antigen-binding region, and an Fc portion including a first Fc peptide fragment and a second Fc peptide fragment. The first antigen-binding region includes an scFv fragment and a binding protein or fragment thereof of a first molecule. The binding protein of the first molecule or the fragment thereof is linked to an N-terminus of the scFv fragment, and a C-terminus of the scFv fragment is linked to an N-terminus of the first Fc peptide fragment. The second antigen-binding region includes a binding protein or fragment thereof of a second molecule. The binding protein or fragment thereof of the second molecule is linked to an N-terminus of the second Fc peptide fragment. The first molecule and the second molecule are identical, and the scFv fragment has CD3 binding activity.

The bispecific antibody according to the embodiments of the present disclosure can bind to CD3 and a tumor target and has low CD3 binding activity and a weak capability to bridge T cells and tumor cells. At the same time, it can effectively promote an activity of peripheral blood mononuclear cells (PBMCs) for killing tumors, has enhanced T cell activation performance, and can promote drug distribution to a tumor site rather than periphery or lymphoid tissues. Thus, the bispecific antibody of the present disclosure has good safety, high anti-cancer activity, and high value in clinical application and drug development.

According to an embodiment of the present disclosure, the first molecule and the second molecule are selected from are each selected from MICA, programmed death ligand 1 (PDL1), CD47, T cell immunoglobulin and ITIM domain (TIGIT), CD73, CD33, carcinoembryonic antigen-related cell adhesion molecule 1 (CEACAM1), CEACAM5, CEACAM6, stimulator of interferon genes (STING), WNT, Beta catenin, B7H3, V-domain Ig suppressor of T cell activation (VISITA), CD19, B-cell maturation antigen (BCMA), CD22, CD20, CD123, CD38, carcinoembryonic antigen (CEA), CD25, CD46, CD138, prostate stem cell antigen (PSCA), prostate-specific membrane antigen (PSMA), prostate-specific antigen (PSA), MUC1, MUC16, New York esophageal squamous cell carcinoma 1 (NY-ESO-1), GD2, WT1, Mesothelin, melanoma-associated Antigen A3 (MAGE-A3), glypican 3 (GPC3), preferentially expressed antigen of melanoma (PRAME), globohexaosylceramide (Globo H), Alpha-fetoprotein (AFP), trophoblast cell surface antigen 2 (Trop2), folate receptor 1 (FOLR1), signal peptide (SP), stem cell antigen 1 (Sca-1), CD133, or epithelial cell adhesion molecule (EPCAM).

According to an embodiment of the present disclosure, both the first molecule and the second molecule are MICA. The bispecific antibody of the present disclosure can bind to CD3 and MICA and has low CD3 binding activity and a weak capability to bridge T cells and tumor cells. Thus, the bispecific antibody has low binding activity to T cells and high binding activity to tumors, and can effectively promote PBMC-mediated killing of tumor cells as well as drug distribution to a tumor site rather than periphery or lymphoid tissues. Thus, the bispecific antibody has good safety and high anti-cancer activity.

According to an embodiment of the present disclosure, the binding protein or fragment thereof is selected from at least one of an antibody or an antigen-binding fragment thereof, a receptor, or a ligand.

According to an embodiment of the present disclosure, the binding protein or fragment thereof of the first molecule is a first Fab fragment; and/or the binding protein or fragment thereof of the second molecule is a second Fab fragment.

It should be noted that all the amino acid sequences involved in the present disclosure are all shown from the N-terminus to the C-terminus.

According to an embodiment of the present disclosure, a C-terminus of a CH1 fragment of the first Fab fragment is linked to the N-terminus of the scFv fragment, and the C-terminus of the scFv fragment is linked to the N-terminus of the first Fc peptide fragment; and/or a C-terminus of a CH1 fragment of the second Fab fragment is linked to the N-terminus of the second Fc peptide fragment.

In some embodiments of the present disclosure, the binding protein or fragment thereof of the first molecule is a first scFab fragment; and/or the binding protein or fragment thereof of the second molecule is a second scFab fragment.

In some embodiments of the present disclosure, a C-terminus of a CH1 fragment of the first scFab fragment is linked to the N-terminus of the scFv fragment, and the C-terminus of the scFv fragment is linked to the N-terminus of the first Fc peptide fragment; and/or a C-terminus of a CH1 fragment of the second scFab fragment is linked to the N-terminus of the second Fc peptide fragment.

For the bispecific antibody according to the embodiments of the present disclosure, its scFv fragment is successfully masked by the first Fab fragment or the first scFab fragment in conformation. Thus, the bispecific antibody exhibits a reduced CD3 binding activity, a weakened ability to bridge T cells and tumor cells, and has good safety. Unexpectedly to the inventors, tumor cell-binding activity of the bispecific antibody with this conformation is significantly enhanced.

According to an embodiment of the present disclosure, both the first Fab fragment and the second Fab fragment have MICA binding activity. For the bispecific antibody according to the embodiments of the present disclosure, its CD3 antigen-binding portion has little effect on affinity of a MICA antigen-binding portion. Therefore, in order to obtain bispecific antibodies with good safety and high anti-cancer activity, it is preferable that an antigen-binding fragment of an anti-MICA antibody with strong MICA binding ability is selected for the MICA antigen-binding portion. For example, a MICA antigen-binding region is selected from a antigen-binding fragment Fab of an h5A1002 antibody.

In some embodiments of the present disclosure, the first Fab fragment and the second Fab fragment are different.

In some embodiments of the present disclosure, the first Fab fragment and the second Fab fragment are identical.

According to an embodiment of the present disclosure, both the first Fab fragment and the second Fab fragment include second heavy chain variable regions CDR1 to CDR3 and second light chain variable regions CDR1 to CDR3, wherein:
the second heavy chain variable region CDR1 has an amino acid sequence as set forth in SEQ ID NO: 7 or a conservative modification form thereof;
the second heavy chain variable region CDR2 has an amino acid sequence as set forth in SEQ ID NO: 8 or a conservative modification form thereof;
the second heavy chain variable region CDR3 has an amino acid sequence as set forth in SEQ ID NO: 9 or a conservative modification form thereof;
the second light chain variable region CDR1 has an amino acid sequence as set forth in SEQ ID NO: 10 or a conservative modification form thereof;
the second light chain variable region CDR2 has an amino acid sequence as set forth in SEQ ID NO: 11 or a conservative modification form thereof; and
the second light chain variable region CDR3 has an amino acid sequence as set forth in SEQ ID NO: 12 or a conservative modification form thereof.

According to the embodiments of the present disclosure, the bispecific antibody of the present disclosure not only has low CD3 binding activity and a weak capability to bridge T cells and tumor cells, and high tumor cell-binding activity, but also can significantly promoting PBMC-mediated killing of tumor cells.

It should be noted that one or more amino acid residues in the heavy chain variable region CDR sequence or the light chain variable region CDR sequence of the present disclosure may be replaced with other amino acid residues from the same side chain family, and the retained function of the modified antibody can be tested for using the functional assays described herein. Preferably, the number of conservative modifications does not exceed one or two.

According to an embodiment of the present disclosure, the first Fab fragment and/or the second Fab fragment includes a second heavy chain framework region and/or a second light chain framework region.

According to an embodiment of the present disclosure, at least a portion of the second heavy chain framework region and/or the second light chain framework region is from at least one of a murine antibody, a humanized antibody, a primate-derived antibody, a bovine-derived antibody, an equine-derived antibody, a dairy bovine-derived antibody, a porcine-derived antibody, a sheep-derived antibody, a goat-derived antibody, a canine-derived antibody, a feline-derived antibody, a rabbit-derived antibody, a camel-derived antibody, a donkey-derived antibody, a deer-derived antibody, a mink-derived antibody, a chicken-derived antibody, a duck-derived antibody, a goose-derived antibody, a turkey-derived antibody, a gamecock-derived antibody, or a mutant thereof, and preferably, from at least one of a murine antibody, a humanized antibody, or a primate-derived antibody.

According to an embodiment of the present disclosure, the first Fab fragment and the second Fab fragment each independently include: a second heavy chain variable region having an amino acid sequence as set forth in SEQ ID NO: 29 or a conservative modification form thereof; and a second light chain variable region having an amino acid sequence as set forth in SEQ ID NO: 30 or a conservative modification form thereof.

According to an embodiment of the present disclosure, the first Fab fragment and/or the second Fab fragment further includes a CH1 fragment and a CL fragment, wherein: a C-terminus of a second heavy chain variable region is linked to an N-terminus of the CH1 fragment; a C-terminus of a second light chain variable region is linked to an N-terminus of the CL fragment; and the CH1 fragment is linked to the CL fragment via an interchain disulfide bond.

According to an embodiment of the present disclosure, the first Fab fragment and the second Fab fragment each independently include: a second heavy chain variable region and a CH1 fragment, having an amino acid sequence as set forth in SEQ ID NO: 31; and a second light chain variable region and a CL fragment, having an amino acid sequence as set forth in SEQ ID NO: 32.

According to an embodiment of the present disclosure, the scFv fragment includes first heavy chain variable regions CDR1 to CDR3 and first light chain variable regions CDR1 to CDR3, wherein:
the first heavy chain variable region CDR1 has an amino acid sequence as set forth in SEQ ID NO: 1;
the first heavy chain variable region CDR2 has an amino acid sequence as set forth in SEQ ID NO: 2;
the first heavy chain variable region CDR3 has an amino acid sequence as set forth in SEQ ID NO: 3;
the first light chain variable region CDR1 has an amino acid sequence as set forth in SEQ ID NO: 4;
the first light chain variable region CDR2 has an amino acid sequence as set forth in SEQ ID NO: 5; and
the first light chain variable region CDR3 has an amino acid sequence as set forth in SEQ ID NO: 6.

According to the embodiments of the present disclosure, when the scFv fragment of the bispecific antibody of the present disclosure has the amino acid sequences of the CDRs described above, the bispecific antibody has significantly reduced CD3 binding activity and a significantly enhanced tumor cell binding capability. Moreover, the bispecific antibody can further promote PBMC-mediated killing of tumor cells. Thus, anti-cancer efficacy and safety can be further improved.

According to an embodiment of the present disclosure, the scFv fragment includes a first heavy chain frame region and/or a first light chain frame region.

According to an embodiment of the present disclosure, at least a portion of the first heavy chain framework region and/or the first light chain framework region is from at least one of a murine antibody, a humanized antibody, a primate-derived antibody, a bovine-derived antibody, an equine-derived antibody, a dairy bovine-derived antibody, a porcine-derived antibody, a sheep-derived antibody, a goat-derived antibody, a canine-derived antibody, a feline-derived antibody, a rabbit-derived antibody, a camel-derived antibody, a donkey-derived antibody, a deer-derived antibody, a mink-derived antibody, a chicken-derived antibody, a duck-derived antibody, a goose-derived antibody, a turkey-derived antibody, a gamecock-derived antibody, or a mutant thereof, and preferably, from at least one of a murine antibody, a humanized antibody, and a primate-derived antibody.

It should be noted that the immunoglobulin described herein may be an immunoglobulin molecule of any type (e.g., IgG, IgE, IgM, IgD, and IgA), class (e.g., IgG1, IgG2, IgG3, IgG4, IgA1, and IgA2), or subclass, including engineered subclasses with altered Fc portions that provide reduced or enhanced effector cell activity. The immunoglobulin may be from any species.

According to an embodiment of the present disclosure, the scFv fragment includes: a first heavy chain variable region having an amino acid sequence as set forth in SEQ ID NO: 33 or a conservative modification form thereof; and a first light chain variable region having an amino acid sequence as set forth in SEQ ID NO: 34 or a conservative modification form thereof.

It should be noted that, one or more amino acid residues in the heavy chain variable region or the light chain variable region of the present disclosure may be replaced with other amino acid residues from the same side chain family, and the retained function of the modified antibody can be tested using the functional assays described herein. Preferably, the number of conservative modifications does not exceed one or two.

According to an embodiment of the present disclosure, the scFv fragment further includes a linker peptide 1, wherein: an N-terminus of the linker peptide 1 is linked to a C-terminus of the first heavy chain variable region, and a C-terminus of the linker peptide 1 is linked to an N-terminus of the first light chain variable region; or the N-terminus of the linker peptide 1 is linked to a C-terminus of the first light chain variable region, and the C-terminus of the linker peptide 1 is linked to an N-terminus of the first heavy chain variable region.

The linker peptide 1 has an amino acid sequence as set forth in (GGGGS)n, where n is an integer greater than or equal to 1, and preferably, 1, 2, 3, 4, 5, 6, 7, 8, 9, or 10.

In some embodiments of the present disclosure, the linker peptide 1 has an amino acid sequence as set forth in SEQ ID NO: 11.

According to an embodiment of the present disclosure, the scFv fragment has an amino acid sequence as set forth in SEQ ID NO: 36 or SEQ ID NO: 37.

According to an embodiment of the present disclosure, the first antigen-binding region further includes a linker peptide 2, wherein a C-terminus of a CH1 fragment of a first Fab fragment is linked to an N-terminus of the linker peptide 2; and a C-terminus of the linker peptide 2 is linked to the N-terminus of the scFv fragment.

According to an embodiment of the present disclosure, the linker peptide 2 has an amino acid sequence as set forth in (GGGGS)n, where n is an integer greater than or equal to 1, and preferably, 1, 2, 3, 4, 5, 6, 7, 8, 9, or 10.

According to an embodiment of the present disclosure, the linker peptide 2 has an amino acid sequence as set forth in SEQ ID NO: 36 or SEQ ID NO: 37.

According to an embodiment of the present disclosure, the first Fc peptide fragment and the second Fc peptide fragment are linked via a knock-into-hole structure.

According to an embodiment of the present disclosure, both the first Fc peptide fragment and the second Fc peptide fragment are human Fc peptide fragments.

According to an embodiment of the present disclosure, the human Fc peptide fragment is a human IgG1 Fc peptide fragment.

According to an embodiment of the present disclosure, the first Fc peptide fragment has an amino acid sequence as set forth in SEQ ID NO: 38; and the second Fc peptide fragment has an amino acid sequence as set forth in SEQ ID NO: 39 or SEQ ID NO: 40.

According to an embodiment of the present disclosure, the bispecific antibody includes:
a first polypeptide chain having an amino acid sequence as set forth in SEQ ID NO: 13, SEQ ID NO: 18, SEQ ID NO: 19, SEQ ID NO: 22, SEQ ID NO: 25, or SEQ ID NO: 28 or an amino acid sequence having at least 80% identity thereto;
a second polypeptide chain having an amino acid sequence as set forth in SEQ ID NO: 14, SEQ ID NO: 20, SEQ ID NO: 23, or SEQ ID NO: 26 or an amino acid sequence having at least 80% identity thereto;
a third polypeptide chain having an amino acid sequence as set forth in SEQ ID NO: 15, SEQ ID NO: 21, SEQ ID NO: 24, or SEQ ID NO: 27 or an amino acid sequence having at least 80% identity thereto; and
a fourth polypeptide chain having an amino acid sequence as set forth in SEQ ID NO: 15, SEQ ID NO: 21, SEQ ID NO: 24, or SEQ ID NO: 27 or an amino acid sequence having at least 80% identity thereto.

It should be noted that, without substantially affecting the binding activity of the multispecific antibody CD3 and MICA (retaining at least 95% of activity), those skilled in the art can substitute, add, and/or delete one or more (e.g., 1, 2, 3, 4, 5, 6, 7, 8, 9, or 10 or more) amino acids in the amino acid sequence of the polypeptide chain included in the multispecific antibody of the present disclosure, to obtain an variant of the amino acid sequence of the polypeptide chain of the bispecific antibody, which are considered to be included within the scope of the present disclosure. For example, amino acids having similar properties in the polypeptide chain may be substituted. The amino acid sequence of the above-mentioned variant of the present disclosure may have at least 80%, 85%, 90%, 95%, 96%, 97%, 98% or 99% identity (or homology) to the reference amino acid sequence.

According to an embodiment of the present disclosure, the bispecific antibody includes:
a first polypeptide chain having an amino acid sequence as set forth in SEQ ID NO: 13, a second polypeptide chain having an amino acid sequence as set forth in SEQ ID NO: 14, a third polypeptide chain having an amino acid sequence as set forth in SEQ ID NO: 15, and a fourth polypeptide chain having an amino acid sequence as set forth in SEQ ID NO: 15; or
a first polypeptide chain having an amino acid sequence as set forth in SEQ ID NO: 18, a second polypeptide chain having an amino acid sequence as set forth in SEQ ID NO: 14, a third polypeptide chain having an amino acid sequence as set forth in SEQ ID NO: 15, and a fourth polypeptide chain having an amino acid sequence as set forth in SEQ ID NO: 15; or
a first polypeptide chain having an amino acid sequence as set forth in SEQ ID NO: 19, a second polypeptide chain having an amino acid sequence as set forth in SEQ ID NO: 14, a third polypeptide chain having an amino acid sequence as set forth in SEQ ID NO: 15, and a fourth polypeptide chain having an amino acid sequence as set forth in SEQ ID NO: 15; or
a first polypeptide chain having an amino acid sequence as set forth in SEQ ID NO: 22, a second polypeptide chain having an amino acid sequence as set forth in SEQ ID NO: 20, a third polypeptide chain having an amino acid sequence as set forth in SEQ ID NO: 21, and a fourth polypeptide chain having an amino acid sequence as set forth in SEQ ID NO: 21; or
a first polypeptide chain having an amino acid sequence as set forth in SEQ ID NO: 25, a second polypeptide chain having an amino acid sequence as set forth in SEQ ID NO: 23, a third polypeptide chain having an amino acid sequence as set forth in SEQ ID NO: 24, and a fourth polypeptide chain having an amino acid sequence as set forth in SEQ ID NO: 24; or
a first polypeptide chain having an amino acid sequence as set forth in SEQ ID NO: 28, a second polypeptide chain having an amino acid sequence as set forth in SEQ ID NO: 26, a third polypeptide chain having an amino acid sequence as set forth in SEQ ID NO: 27, and a fourth polypeptide chain having an amino acid sequence as set forth in SEQ ID NO: 27.

### Nucleic Acid

The present disclosure provides a nucleic acid. The nucleic acid encodes the aforementioned antibody or the antigen-binding fragment thereof. The nucleic acid molecule encodes the aforementioned bispecific antibody. The nucleic acid molecule according to the embodiments of the present disclosure can encode a bispecific antibody that can simultaneously target CD3 and a tumor target.

It should be noted that those skilled in the art should understand that the nucleic acid molecules mentioned in the present disclosure actually include any one or two of complementary double strands. For convenience, in the present disclosure, although only one chain is given in most cases, the other chain complementary thereto is actually also disclosed. In addition, the sequence of the molecule in the present disclosure includes a DNA form or an RNA form. When one form is disclosed, it means that the other form is also disclosed.

### Vector or Transformant

The present disclosure provides a vector or transformant. The vector or transformant includes the above-mentioned nucleic acid. The vector or transformant according to the embodiments of the present disclosure may include an optional control sequence. The control sequence is operably linked to the nucleic acid molecule. The control sequence refers to one or more control sequences that can direct the expression of the nucleic acid molecule in the host. Thus, the constructed vector or transformant can effectively express the aforementioned bispecific antibody.

When the above-mentioned nucleic acid molecule is linked to the vector or transformant, such as an expression vector, the nucleic acid molecule may be directly or indirectly linked to the control components on the expression vector, as long as these control components can control translation and expression of the nucleic acid molecule. Of course, these control components may be directly derived from the vector, and they may also be exogenous, that is, not from the vector itself. The nucleic acid molecule is operatively linked to the control element.

According to an embodiment of the present disclosure, the vector may refer to a cloning vector or an expression vector, and can be obtained by operatively linking the nucleic acid to a commercially available vector (such as a plasmid or viral vector). The vector in the present disclosure is not particularly limited, and common plasmids such as pSeTag2, PEE14, and pMH3 can be used.

In the present disclosure, the term "operable linked" means that an exogenous gene is linked to a vector, enabling control elements in the vector, such as a transcriptional control amino acid sequence and a translational control amino acid sequence, to exert an expected effect of regulating transcription and translation of the exogenous gene. Commonly used vectors may be, for instance,viral vectors, plasmids, bacteriophages, etc. After the expression vector according to some specific embodiments of the present disclosure is introduced into suitable recipient cells, the expression of the aforementioned nucleic acid molecule can be effectively realized under the mediation of the regulatory system, thereby enabling the *in vitro* large-scale generation of proteins encoded by the nucleic acid molecule.

According to an embodiment of the present disclosure, the vector is a eukaryotic vector or a prokaryotic vector.

According to an embodiment of the present disclosure, the vector includes at least one selected from a plasmid vector, an adenoviral vector, a lentiviral vector, or an adeno-associated viral vector.

### Cell

The present disclosure provides a cell. The cell carries the aforementioned nucleic acid or the aforementioned vector or transformant, or expresses the aforementioned bispecific antibody. The cell according to the embodiments of the present disclosure is obtained by transfection or transformation of the vector or transformant, and can thus efficiently express the aforementioned bispecific antibody under a suitable condition.

According to an embodiment of the present disclosure, the cell is a prokaryotic cell, a eukaryotic cell, or a bacteriophage.

According to an embodiment of the present disclosure, the prokaryotic cell is *Escherichia coli, Bacillus subtilis, Streptomyces,* or *Proteus mirabilis.*

According to an embodiment of the present disclosure, the eukaryotic cell is fungus, an insect cell, a plant cell, or a mammalian cell.

According to an embodiment of the present disclosure, the fungus is *Pichia pastoris, Saccharomyces cerevisiae, Schizosaccharomyces pombe,* or *Trichoderma.*

According to an embodiment of the present disclosure, the insect cell is a *Spodoptera frugiperda* cell. According to an embodiment of the present disclosure, the plant cell is *Nicotiana tabacum* cell. According to an embodiment of the present disclosure, the mammalian cell is a BHK cell, a CHO cell, a COS cell, a myeloma cell, or a human embryonic kidney 293 cell, and does not include an animal germ cell, fertilized egg, or embryonic stem cell.

According to an embodiment of the present disclosure, the cells are mammalian cells.

According to an embodiment of the present disclosure, the cells are BHK cells, CHO cells, COS cells, or NSO cells.

It should be noted that "suitable conditions" as used in the description of the present application refers to conditions suitable for the expression of the antibody or the antigen binding fragment thereof described herein. Those skilled in the art will readily appreciate that conditions suitable for the expression of the antibody or antigen binding fragment include, but are not limited to, suitable transformation or transfection manners, suitable transformation or transfection conditions, healthy host cell state, suitable host cell density, suitable cell culture environment, and suitable cell culture time. The "suitable conditions" are not particularly limited, and one skilled in the art can optimize the optimal conditions for expression of the recombinant antibody according to the particular circumstances of the laboratory.

### Pharmaceutical Composition

The present disclosure provides a pharmaceutical composition. The pharmaceutical composition includes the aforementioned bispecific antibody, the aforementioned nucleic acid, the aforementioned vector or transformant, or the aforementioned cell. The bispecific antibody according to the embodiments of the present disclosure can effectively promote PBMC-mediated killing of tumor cells and has high anti-cancer activity. Moreover, it has low CD3 binding activity and good safety. Thus, the resulting medicament can be further used to prevent and/or treat a CD3-mediated disease and/or a tumor target-mediated disease.

According to an embodiment of the present disclosure, the pharmaceutical composition further includes a pharmaceutically acceptable adjuvant.

According to an embodiment of the present disclosure, the adjuvant includes one or more pharmaceutically acceptable excipients, diluents, stabilizers, or carriers.

According to an embodiment of the present disclosure, the pharmaceutical composition is in the form of an injection.

It should be noted that the pharmaceutical composition includes combinations that are separated in time and/or space as long as they can work together to achieve the purpose of the present disclosure. For example, the components contained in the composition can be administered to subjects as a whole or separately. When the components contained in the composition are administered separately to a subject, the individual components may be administered to the subject simultaneously or sequentially.

The medicament according to the present disclosure includes a safe and effective amount of active ingredients of the present disclosure and pharmaceutically acceptable adjuvants. These adjuvants include but not limited to: saline, buffer solution, glucose, water, glycerin, ethanol and combinations thereof. Generally, pharmaceutical preparations should be matched with an administration mode. Dosage forms of the medicament according to the present disclosure include injection, oral preparation (tablets, capsules and oral liquid), transdermal preparations, and sustained-release preparations. For example, the medicament is prepared with normal saline or an aqueous solution containing glucose and other adjuvants by a conventional method. The medicament is suitable to be prepared under a sterile condition.

The effective amount of the active ingredient according to the present disclosure may vary with a mode of administration and severity of the to-be-treated disease. Preferably, the effective amount may be determined by those skilled in the art based on various factors (such as though a clinical test). The factors include, but not limited to: pharmacokinetic parameters of active ingredients, for example, bioavailability, metabolism, and half-life period; severity of the to-be-treated diseases of patients; weight of the patient; immune states of the patient; and route of administration. For example, based on the urgent requirements of treatment states, separated doses may be administrated several times per day, or the dose can be reduced proportionally.

The pharmaceutically acceptable adjuvants according to the present disclosure include but is not limited to water, saline, liposomes, lipids, proteins, protein-antibody conjugates, peptides, cellulose, nanogels, or combinations thereof. The selection of the carrier should be matched with should be matched with an administration mode, as is well known to those skilled in the art.

### Kit

The present disclosure provides a kit. The kit includes the aforementioned bispecific antibody, the aforementioned nucleic acid molecule, the aforementioned vector or transformant, or the aforementioned cell. The kit according to the embodiments of the present disclosure can bind to a CD3 protein and/or a tumor target, and can thus effectively identify CD3 protein and/or tumor targets.

Those skilled in the art will understand that the features and advantages described above for the antibody, the nucleic acid molecule, the vector or transformant, the cell, and the pharmaceutical composition are applicable to the kit, which is not described in detail herein.

In some embodiments of the present disclosure, the tumor target is MICA. As previously described, the bispecific antibody according to the embodiments of the present disclosure can specifically bind to CD3 and MICA. CD3 protein and/or MICA protein-related kits developed utilizing this property can be used for research related to a CD3 protein and/or a MICA protein, such as detection and/or enrichment and/or separation and purification of CD3 and/or MICA proteins of humans or other mammals. The kit can effectively detect, enrich, or separate and purify CD3 and/or MICA proteins in biological samples for further scientific research, such as qualitative or quantitative detection of CD3 and/or MICA protein molecules in biological samples. More specifically, it can be used in kits for detection involving the specific binding properties of CD3 protein and/or MICA protein to antibodies, such as immunoblotting, and immunoprecipitation. These kits may include any one or more of the following: antagonist, the bispecific antibody of the present disclosure, or pharmaceutical reference material; a protein purification column; an immunoglobulin affinity purification buffer; an assay diluent for cells. The bispecific antibody of the present disclosure can be used for different types of diagnostic tests, e.g. in vitro or in vivo detection of the presence of a wide variety of diseases or medicaments, toxins or other proteins, etc. For example, CD3 and/or MICA-related diseases can be tested by testing the serum or blood of subject.

### Use in Manufacture of Kit

The present disclosure provides use of the aforementioned bispecific antibody, the aforementioned nucleic acid molecule, the aforementioned vector or transformant, or the aforementioned cell in the manufacture of a kit for detecting CD3 and/or a tumor target.

In some embodiments of the present disclosure, the tumor target is MICA.

Those skilled in the art will understand that the features and advantages described above for the antibody, the nucleic acid, the vector or transformant, the cell, or the pharmaceutical composition are applicable to the use in the manufacture of the kit, and details thereof are omitted herein.

### Use in Manufacture of Medicament

The present disclosure provides a use of the aforementioned bispecific antibody, the aforementioned nucleic acid molecule, the aforementioned vector or transformant, the aforementioned cell, or the aforementioned pharmaceutical composition in preparation of a medicament for preventing and/or treating a CD3-mediated disease and/or a tumor target-mediated disease.

Those skilled in the art will understand that the features and advantages described above for the antibody, the nucleic acid molecule, the vector or transformant, the cell, and the pharmaceutical composition are applicable to the use in the manufacture of the medicament, and details thereof are omitted herein.

According to an embodiment of the present disclosure, the CD3-mediated related disease includes an autoimmune disease.

According to embodiments of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, bullous pemphigoid, primary biliary cirrhosis, multiple sclerosis, or acute idiopathic polyneuritis.

According to an embodiment of the present disclosure, the tumor target is MICA. The bispecific antibody, the nucleic acid, the vector or transformant, or the pharmaceutical composition according to the embodiments of the present disclosure can be further prepared into a medicament that can be used clinically for prevention or treatment of a CD3 and/or MICA-mediated disease.

According to an embodiment of the present disclosure, the MICA-mediated related disease is a cancer, a disease caused by transplant rejection, an autoimmune disease, or an infectious disease.

According to an embodiment of the present disclosure, the cancer is at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head-and-neck cancer.

### Method for Treating Disease

The present disclosure provides a method for preventing and/or treating a CD3 and/or MICA-mediated disease. According to an embodiment of the present disclosure, the method includes: administering to a subject a pharmaceutically acceptable amount of the aforementioned bispecific antibody, the aforementioned nucleic acid molecule, the aforementioned vector or transformant, the aforementioned cell, or the aforementioned pharmaceutical composition.

It should be noted that the terms "subject," "individual," and "patient" may be used interchangeably herein and refer to a mammal being evaluated for treatment and/or being treated. In an embodiment, the mammal is a human. The terms "subject," "individual," and "patient" include, but are not limited to, individuals with cancer, individuals with autoimmune diseases, individuals with pathogen infection, and the like. The subject may be a human, but also includes other mammals, especially mammals that can be used as laboratory models for human diseases, such as mice and rats.

The effective amount of the antibody or the antigen-binding fragment thereof, the nucleic acid, the vector or transformant, or the pharmaceutical composition according to the present disclosure may vary depending on the mode of administration and the severity of the disease to be treated. The selection of the optimal effective amount can be determined by those skilled in the art based on various factors (such as through clinical trials). Such factors include, but are not limited to pharmacokinetic parameters of the active ingredient such as bioavailability, metabolism, half-life, and the like; the severity of the disease to be treated of the patient, the weight of the patient, the immune status of the patient, the route of administration, etc. For example, several divided doses may be administered daily or the dose may be proportionally reduced depending on the exigencies of the therapeutic situation.

In some specific embodiments, the CD3-mediated disease includes an autoimmune disease.

In some specific embodiments, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, bullous pemphigoid, primary biliary cirrhosis, multiple sclerosis, or acute idiopathic polyneuritis.

In some specific embodiments, the tumor target is MICA.

In some specific embodiments, the MICA-mediated disease is a cancer, a disease caused by transplant rejection, an autoimmune disease, or an infectious disease.

In some specific embodiments, the cancer is at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head-and-neck cancer.

The description of the amino acid sequences involved in the present disclosure are detailed in Table 1.

**[Table 1] Amino acid sequence description table**

| SEQ ID NO: | Sequence | Description |
|---|---|---|
| 1 | GFTFNTYA | Cross3 HCDR1 |
| 2 | IRSKYNNYAT | Cross3 HCDR2 |
| 3 | VRHGNFGNSYVSWFAY | Cross3 HCDR3 |
| 4 | TGAVTTSNY | Cross3 LCDR1 |
| 5 | GTN | Cross3 LCDR2 |
| 6 | ALWYSNLWV | Cross3 LCDR3 |
| 7 | GFSLTTYGG | h5A1002 |
| | | HCDR1 |
| 8 | IWTDGWT | h5A1002 |
| | | HCDR2 |
| 9 | ARKGHGYYYAMDY | h5A1002 |
| | | HCDR3 |
| 10 | SSVSSSY | h5A1002 |
| | | LCDR1 |
| 11 | STSNLI | h5A1002 |
| | | LCDR2 |
| 12 | HQYHRSPFT | h5A1002 |
| | | LCDR3 |
| 13 | QVQLQESGPGLVKPSETLSLTCTVSGFSLTTYGGHWIRQPPGKGLEWIG MIWTDGWTDYNAAFISRVTISVDTSKNQFSLKLSSVTAADTAVYYCAR KGHGYYYAMDYWGQGTLVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTSPPSPEVQLLESGGGLV QPGGSLKLSCAASGFTFNTYAMNWVRQAPGKGLEWVARIRSKYNNYA TYYADSVKDRFTISRDDSKNTAYLQMNNLKTEDTAVYYCVRHGNFGNS YVSWFAYWGQGTLVTVSSGGGGSGGGGSGGGGSELVVTQEPSLTVSPG GTVTLTCRSSTGAVTTSNYANWVQQKPGQAPRGLIGGTNKRAPGTPAR FSGSLLGGKAALTLSGVQPEDEAEYYCALWYSNLWVFGGGTKLTVLGG GGSPKSSDKTHTCPPCPAPEAAGGPSVFLFPPKPKDTLMISRTPEVTCVV VDVSHEDPEVKFNWYVDGVEVHNAKTKPREEQYNSTYRVVSVLTVLH QDWLNGKEYKCKVSNKALPAPIEKTISKAKGQPREPQVYTLPPCREEM TKNQVSLWCLVKGFYPSDIAVEWESNGQPENNYKTTPPVLDSDGSFFLY SKLTVDKSRWQQGNVFSCSVMHEALHNHYTQKSLSLSPGK | h5A1002-Cross3-kbss |
| 14 | QVQLQESGPGLVKPSETLSLTCTVSGFSLTTYGGHWIRQPPGKGLEWIG MIWTDGWTDYNAAFISRVTISSVDTSKNQFSLKLSSVTAADTAVYYCAR KGHGYYYAMDYWGQG TLVTVSSASTKGPSVFPLAPSSKSGGTAALGCLVKDYFPEPVTVSWNSG ALTSGVHTFPAVLQSSGLYSLSSVVTVPSSSLGTQTYICNVNHKPSNTKV DKKVEPKSCDK THTCPPCPAPEAAGGPSVFLFPPKPKDTLMISRTPEVTCVVVDVSHEDPE VKFNWYVDGVEVHNAKTKPREEQYNSTYRVVSVLTVLHQDWLNGKE YKCKVSNKALPAPIEK TISKAKGQPREPQVCTLPPSREEMTKNQVSLSCAVKGFYPSDIAVEWES NGQPENNYKTTPPVLDSDGSFFLVSKLTVDKSRWQQGNVFSCSVMHEA LHNHYTQKSLSLSPGK | h5A1002-hoss |
| 15 | EIVLTQSPGTLSLSPGERATLSCTASSSVSSSYLHWYQQKPGQAPRLLIYS TSNLISGIPDRFSGSGSGTDFTLTISRLEPEDFAVYYCHQYHRSPFTFGQG TKLEI KRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSS PVTKSFNRGEC | h5A1002-hK |
| 16 | EVQLLESGGGLVQPGGSLKLSCAASGFTFNTYAMNWVRQAPGKGLEW VARIRSKYNNYATYYADSVKDRFTISRDDSKNTAYLQMNNLKTEDTAVY YCVRHGNFGNSYVSWFAYWGQGTLV TVSSGGGGSGGGGSGGGGSELVVTQEPSLTVSPGGTVTLTCRSSTGAVT TSNYANWVQQKPGQAPRGLIGGTNKRAPGTPARFSGSLLGGKAALTLS GVQPEDEAEYYCALWYSNLWVFGG GTKLTVLGGGGSPKSSDKTHTCPPCPAPEAAGGPSVFLFPPKPKDTLMIS RTPEVTCVVVDVSHEDPEVKFNWYVDGVEVHNAKTKPREEQYNSTYR VVSVLTVLHQDWLNGKEYKCKVSN KALPAPIEKTISKAKGQPREPQVYTLPPCREEMTKNQVSLWCLVKGFYP SDIAVEWESNGQPENNYKTTPPVLDSDGSFFLYSKLTVDKSRWQQGNVF SCSVMHEALHNHYTQKSLSLSPGK | Cross3-kbss |
| 17 | EVQLVESGGGLVQPGGSLRLSCAASGYSFTGYTMNWVRQAPGKGLEW VALINPYKGVSTYNQKFKDRFTISVDKSKNTAYLQMNSLRAEDTAVYYC ARSGYYGDSDWYFDVWGQGTLVTV SSGGGGSGGGGSGGGGSDIQMTQSPSSLSASVGDRVTITCRASQDIRN YLNWYQQKPGKAPKLLIYYTSRLESGVPSRFSGSGSGTDYTLTISSLQPE DFATYYCQQGNTLPWTFGQGTK VEIKGGGGSPKSSDKTHTCPPCPAPEAAGGPSVFLFPPKPKDTLMISRTP EVTCVVVDVSHEDPEVKFNWYVDGVEVHNAKTKPREEQYNSTYRVVS VLTVLHQDWLNGKEYKCKVSNKA LPAPIEKTISKAKGQPREPQVYTLPPCREEMTKNQVSLWCLVKGFYPSDI AVEWESNGQPENNYKTTPPVLDSDGSFFLYSKLTVDKSRWQQGNVFSC SVMHEALHNHYTQKSLSLSPGK | UCHT1-kbss |
| 18 | QVQLQESGPGLVKPSETLSLTCTVSGFSLTTYGGHWIRQPPGKGLEWIG MIWTDGWTDYNAAFISRVTISVDTSKNQFSLKLSSVTAADTAVYYCAR KGHGYYYAMDYWGQGTLVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTSPPSPEVQLVESGGGLV QPGGSLRLSCAASGYSFTGYTMNWVRQAPGKGLEWVALINPYKGVST YNQKFKDRFTISVDKSKNTAYLQMNSLRAEDTAVYYCARSGYYGDSD WYFDVWGQGTLVTVSSGGGGSGGGGSGGGGSDIQMTQSPSSLSASVG DRVTITCRASQDIRNYLNWYQQKPGKAPKLLIYYTSRLESGVPSRFSGS GSGTDYTLTISSLQPEDFATYYCQQGNTLPWTFGQGTKVEIKGGGGSPK SSDKTHTCPPCPAPEAAGGPSVFLFPPKPKDTLMISRTPEVTCVVVDVSH EDPEVKFNWYVDGVEVHNAKTKPREEQYNSTYRVVSVLTVLHQDWL NGKEYKCKVSNKALPAPIEKTISKAKGQPREPQVYTLPPCREEMTKNQV SLWCLVKGFYPSDIAVEWESNGQPENNYKTTPPVLDSDGSFFLYSKLTV DKSRWQQGNVFSCSVMHEALHNHYTQKSLSLSPGK | h5A1002-UCHT1-kbss |
| 19 | QVQLQESGPGLVKPSETLSLTCTVSGFSLTTYGGHWIRQPPGKGLEWIG MIWTDGWTDYNAAFISRVTISVDTSKNQFSLKLSSVTAADTAVYYCAR KGHGYYYAMDYWGQGTLVTVSSASTKGPSVFPLAPSSKSTSGGTAALG CLVKDYFPEPVTVSWNSGALTSGVHTFPAVLQSSGLYSLSSVVTVPSSSL GTQTYICNVNHKPSNTKVDKKVEPKSCDKTHTSPPSPQAVVTQEPSLTV SPGGTVTLTCRSSTGAVTTSNYANWVQQKPGQAPRGLIGGTNKRAPWT PARFSGSLLGDKAALTITGAQAEDEAEYFCALWYSNLWVFGGGTKLTV LGGSGGSGGSGGSGGSGGSGGSGGSEVQLVESGGGLVQPGGSLKLSCA ASGFTFNTYAMNWVRQAPGKGLEWVARIRSKYNNYATYYADSVKDRF TISRDDSKSTLYLQMNSLKTEDTAVYYCVRHGNFGNSYVSWFAYWGQG TLVTVSSGGGGSPKSSDKTHTCPPCPAPEAAGGPSVFLFPPKPKDTLMIS RTPEVTCVVVDVSHEDPEVKFNWYVDGVEVHNAKTKPREEQYNSTYR VVSVLTVLHQDWLNGKEYKCKVSNKALPAPIEKTISKAKGQPREPQVY TLPPCREEMTKNQVSLWCLVKGFYPSDIAVEWESNGQPENNYKTTPPVL DSDGSFFLYSKLTVDKSRWQQGNVFSCSVMHEALHNHYTQKSLSLSPG K | h5A1002-SP34-kbss |
| 20 | EIQLVQSGAEVKKPGASVKVSCKASGYAFTSQNIYWVRQAPGQGLEWI GYIEPYNVVPMYNPKFKGRATLTVDKSTSTAYLELSSLRSEDTAVYYCA RSGSSNFDYWGQGT LVTVSSASTKGPSVFPLAPSKSTSGGTAALGCLVKDYFPEPVTVSWNSG ALTSGVHTFPAVLQSSGLYSLSSVVTVPSSSLGTQTYICNVNHKPSNTKV DKKVEPKSCDKT HTCPPCPAPEAAGGPSVFLFPPKPKDTLMISRTPEVTCVVVDVSHEDPEV KFNWYVDGVEVHNAKTKPREEQYNSTYRVVSVLTVLHQDWLNGKEY KCKVSNKALPAPIEKT ISKAKGQPREPQVCTLPPSREEMTKNQVSLSCAVKGFYPSDIAVEWESN GQPENNYKTTPPVLDSDGSFFLVSKLTVDKSRWQQGNVFSCSVMHEAL HNHYTQKSLSLSPGK | 1D5-hoss |
| 21 | DIQLTQSPSSLSASVGDRVTITCSASSSISSHYLHWYQQKPGKSPKLLIY RTSNLASGVPSRFSGSGSGTDYTLTISSLQPEDFATYYCQQGSSLPLTFGQ GTKVEI KRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSS PVTKSFNRGEC | 1D5-hK |
| 22 | EIQLVQSGAEVKKPGASVKVSCKASGYAFTSQNIYWVRQAPGQGLEWI GYIEPYNVVPMYNPKFKGRATLTVDKSTSTAYLELSSLRSEDTAVYYCA RSGSSNFDYWGQGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKVEPKSCDKTHTSPPSPEVQLLESGGGLVQPG GSLKLSCAASGFTFNTYAMNWVRQAPGKGLEWVARIRSKYNNYATYY ADSVKDRFTISRDDSKNTAYLQMNNLKTEDTAVYYCVRHGNFGNSYVS WFAYWGQGTLVTVSSGGGGSGGGGSGGGGSELVVTQEPSLTVSPGGTV TLTCRSSTGAVTTSNYANWVQQKPGQAPRGLIGGTNKRAPGTPARFSGS LLGGKAALTLSGVQPEDEAEYYCALWYSNLWVFGGGTKLTVLGGGGS PKSSDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPCREEMTKNQ VSLWCLVKGFYPSDIAVEWESNGQPENNYKTTPPVLDSDGSFFLYSKLT VDKSRWQQGNVFSCSVMHEALHNHYTQKSLSLSPGK | 1D5-Cross3-kbss |
| 23 | EVQLVQSGAEVKKPGASVKVSCKASGYAFTNYLIEWVRQAPGQGLEWI GAINPGSGATNYNEKFKDRATLTADKSTNTAYLELSSLRSEDTAVYFCAR FLGNYFDNWGQGT LVTVSSASTKGPSVFPLAPSKSTSGGTAALGCLVKDYFPEPVTVSWNSG ALTSGVHTFPAVLQSSGLYSLSSVVTVPSSSLGTQTYICNVNHKPSNTKV DKKVEPKSCDKT HTCPPCPAPEAAGGPSVFLFPPKPKDTLMISRTPEVTCVVVDVSHEDPEV KFNWYVDGVEVHNAKTKPREEQYNSTYRVVSVLTVLHQDWLNGKEY KCKVSNKALPAPIEKT ISKAKGQPREPQVCTLPPSREEMTKNQVSLSCAVKGFYPSDIAVEWESN GQPENNYKTTPPVLDSDGSFFLVSKLTVDKSRWQQGNVFSCSVMHEAL HNHYTQKSLSLSPGK | 13A9-hoss |
| 24 | DIQMTQSPSSLSASVGDRVTITCRASGNIHSYLAWYQQKPGKSPKLLV YYAETLADGVPSRFSGSGSGTDYTLTISSLQPEDFATYFCQQFWTTPYTF GQGTKVEIK RTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPV TKSFNRGEC | 13A9-hK |
| 25 | EVQLVQSGAEVKKPGASVKVSCKASGYAFTNYLIEWVRQAPGQGLEWI GAINPGSGATNYNEKFKDRATLTADKSTNTAYLELSSLRSEDTAVYFCAR FLGNYFDNWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKVEPKSCDKTHTSPPSPEVQLLESGGGLVQPGG SLKLSCAASGFTFNTYAMNWVRQAPGKGLEWVARIRSKYNNYATYYA DSVKDRFTISRDDSKNTAYLQMNNLKTEDTAVYYCVRHGNFGNSYVS WFAYWGQGTLVTVSSGGGGSGGGGSGGGGSELVVTQEPSLTVSPGGTV TLTCRSSTGAVTTSNYANWVQQKPGQAPRGLIGGTNKRAPGTPARFSGS LLGGKAALTLSGVQPEDEAEYYCALWYSNLWVFGGGTKLTVLGGGGS PKSSDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAPIEKTISKAKGQPREPQVYTLPPCREEMTKNQ VSLWCLVKGFYPSDIAVEWESNGQPENNYKTTPPVLDSDGSFFLYSKLT VDKSRWQQGNVFSCSVMHEALHNHYTQKSLSLSPGK | 13A9-Cross3-kbss |
| 26 | QVQLVESGGGLVKPGGSLRLSCAASGFTFSNYAMSWIRQAPGKGLEWV SYISPGGDYIYYADSVKGRFTISSRDNAKNSLYLQMNSLRAEDTAVYYC TTDRRHYGSYAMDYWG QGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPS NTKVDKKVEPKSCD KTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGK EYKCKVSNKALPAPIEK TISKAKGQPREPQVCTLPPSREEMTKNQVSLSCAVKGFYPSDIAVEWES NGQPENNYKTTPPVLDSDGSFFLVSKLTVDKSRWQQGNVFSCSVMHEA LHNHYTQKSLSLSPGK | 619-hoss |
| 27 | DIVMTQSPLSLPVTPGEPASISCRSSKSLLHSNLNTYLYWFLQKPGQSPQI LIYRMSNLASGVPDRFSGSGSGTAFTLKISRVEAEDVGVYYCMQHLEYP FTFGPGTKL EIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLS SPVTKSFNRGEC | 619-hK |
| 28 | QVQLVESGGGLVKPGGSLRLSCAASGFTFSNYAMSWIRQAPGKGLEWV SYISPGGDYIYYADSVKGRFTISRDNAKNSLYLQMNSLRAEDTAVYYCT TDRRHYGSYAMDYWGQGTLVTVSSASTKGPSVFPLAPSSKSTSGGTAA LGCLVKDYFPEPVTVSWNSGALTSGVHTFPAVLQSSGLYSLSSVVTVPSS SLGTQTYICNVNHKPSNTKVDKKVEPKSCDKTHTSPPSPEVQLLESGGG LVQPGGSLKLSCAASGFTFNTYAMNWVRQAPGKGLEWVARIRSKYNN YATYYADSVKDRFTISRDDSKNTAYLQMNNLKTEDTAVYYCVRHGNFG NSYVSWFAYWGQGTLVTVSSGGGGSGGGGSGGGGSELVVTQEPSLTVS PGGTVTLTCRSSTGAVTTSNYANWVQQKPGQAPRGLIGGTNKRAPGTP ARFSGSLLGGKAALTLSGVQPEDEAEYYCALWYSNLWVFGGGTKLTVL GGGGSPKSSDKTHTCPPCPAPEAAGGPSVFLFPPKPKDTLMISRTPEVTC VVVDVSHEDPEVKFNWYVDGVEVHNAKTKPREEQYNSTYRVVSVLTV LHQDWLNGKEYKCKVSNKALPAPIEKTISKAKGQPREPQVYTLPPCREE MTKNQVSLWCLVKGFYPSDIAVEWESNGQPENNYKTTPPVLDSDGSFF LYSKLTVDKSRWQQGNVFSCSVMHEALHNHYTQKSLSLSPGK | 619-Cross3-kbss |
| 29 | QVQLQESGPGLVKPSETLSLTCTVSGFSLTTYGGHWIRQPPGKGLEWIG MIWTDGWTDYNAAFISRVTISVDTSKNQFSLKLSSVTAADTAVYYCAR KGHGYYYAMDYWGQGTLVTVSS | h5A1002 VH |
| 30 | EIVLTQSPGTLSLSPGERATLSCTASSSVSSSYLHWYQQKPGQAPRLLIYS TSNLISGIPDRFSGSGSGTDFTLTISRLEPEDFAVYYCHQYHRSPFTFGQG TKLEIK | h5A1002 VL |
| 31 | QVQLQESGPGLVKPSETLSLTCTVSGFSLTTYGGHWIRQPPGKGLEWIG MIWTDGWTDYNAAFISRVTISSVDTSKNQFSLKLSSVTAADTAVYYCAR KGHGYYYAMDYW GQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPS NTKVDKKVE | h5A1002 Fab VH-CH1 |
| 32 | EIVLTQSPGTLSLSPGERATLSCTASSSVSSSYLHWYQQKPGQAPRLLIYS TSNLISGIPDRFSGSGSGTDFTLTISRLEPEDFAVYYCHQYHRSPFTFGQG TKLEI KRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSS PVTKSFNRGEC | h5A1002 Fab VL-CL |
| 33 | EVQLLESGGGLVQPGGSLKLSCAASGFTFNTYAMNWVRQAPGKGLEW VARIRSKYNNYATYYADSVKDRFTISRDDSKNTAYLQMNNLKTEDTAVY YCVRHGNFGNSYVSWFAYWGQGTLVTVSS | Cross3 VH |
| 34 | ELVVTQEPSLTVSPGGTVTLTCRSSTGAVTTSNYANWVQQKPGQAPRGL IGGTNKRAPGTPARFSGSLLGGKAALTLSGVQPEDEAEYYCALWYSNL WVFGGGTKLTVL | Cross3 VL |
| 35 | EVQLLESGGGLVQPGGSLKLSCAASGFTFNTYAMNWVRQAPGKGLEW VARIRSKYNNYATYYADSVKDRFTISRDDSKNTAYLQMNNLKTEDTAVY YCVRHGNFGNSYVSWFAYWGQGTLVTVS SGGGGSGGGGSGGGGSELVVTQEPSLTVSPGGTVTLTCRSSTGAVTTSN YANWVQQKPGQAPRGLIGGTNKRAPGTPARFSGSLLGGKAALTLSGVQ PEDEAEYYCALWYSNLWVFGGGTKLTVL | Cross3 scFv |
| 36 | GGGGS | A linker peptide |
| 37 | GGGGSGGGGSGGGGS | A linker peptide |
| 38 | PKSSDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAP IEKTISKAKGQPREPQVYTLPPCREEMTKNQVSLWCLVKGFYPSDIAVE WESNGQPENNYKTTPPVLDSDGSFFLYSKLTVDKSRWQQGNVFSCSVM HEALHNHYTQKSLSLSPGK | First Fc peptide fragment |
| 39 | PKSCDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAP IEKTISKAKGQPREPQVCTLPSREEMTKNQVSLSCAVKGFYPSDIAVEWE SNGQPENNYKTTPPVLDSDGSFFLVSKLTVDKSRWQQGNVFSCSVMHE ALHNHYTQKSLSLSPGK | Second Fc peptide fragment (when the MICA antibody has a Fab or scFab structure) |
| 40 | PKSSDKTHTCPPCPAPEAAGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPAP IEKTISKAKGQPREPQVCTLPSREEMTKNQVSLSCAVKGFYPSDIAVEWE SNGQPENNYKTTPPVLDSDGSFFLVSKLTVDKSRWQQGNVFSCSVMHE ALHNHYTQKSLSLSPGK | Second Fc peptide fragment (when the MICA antibody has an scFv structure) |

The present disclosure will be described in detail below by way of examples. In the examples or test examples, experimental methods without specifying the specific conditions are performed according to conventional conditions.

The solutions of the present disclosure will be explained in conjunction with the examples. Those skilled in the art will understand that the examples described below are only used to illustrate the present disclosure and should not be considered as limiting the scope of the present disclosure. Procedures in the examples without indicated specific technologies or conditions are conducted according to technologies or conditions described in the document in the art or according to the product specification. The reagents or instruments used without specifying the manufacturers are all conventional products that can be obtained commercially..

### Example 1: Production of Antibody

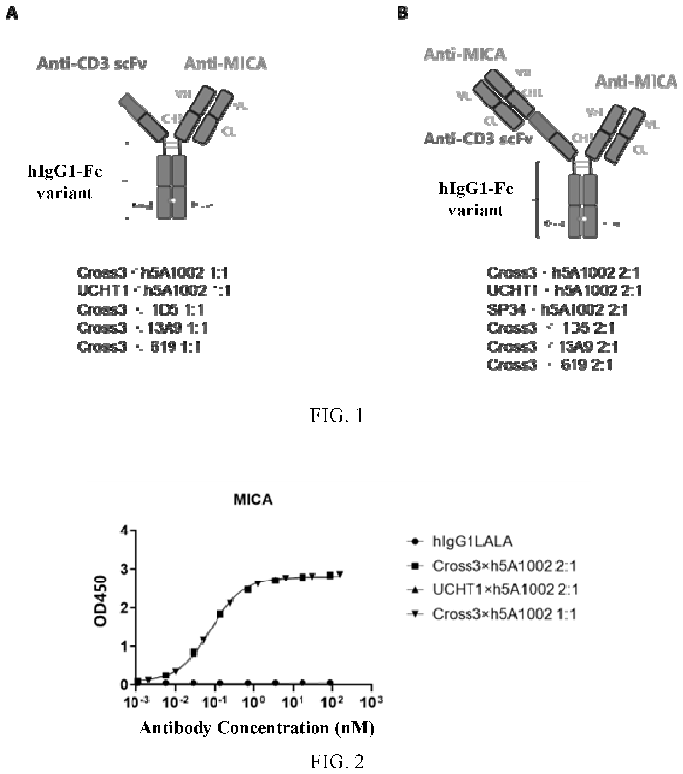

The specific experimental operations for the production of the antibody were as follows: (1) ExpiCHO cells (purchased from Thermo Fisher) were cultured by utilizing an ExpiCHO Expression Medium (purchased from Thermo Fisher), and the cell concentration was adjusted to 6×10⁶/mL to obtain an ExpiCHO cell solution; (2) when the bispecific antibody was in configuration A (FIG. 1), a vector pcDNA3.4 containing a CD3 antibody, a MICA antibody heavy chain, and a MICA antibody light chain (synthesized by commissioned GenScript, Nanjing) was added into 2 mL of OptiSFM medium (purchased from Thermo Fisher) at a ratio of 1:1:1 to obtain a solution a; or when the bispecific antibody was in configuration B (FIG. 1), a vector pcDNA3.4 containing a MICA heavy chain-CD3 antibody, a MICA antibody heavy chain, and a MICA antibody light chain (synthesized by Nanjing GenScript) was added into 2 mL of OptiSFM medium (purchased from Thermo Fisher) at a ratio of 1:1:1 to obtain solution a; (3) 160 µL of an ExpiFectamineCHO transfection reagent (purchased from Thermo Fisher) was added to 2 mL of OptiSFM medium (purchased from Thermo Fisher) to obtain a solution b; (4) then the solution a and the solution b were mixed to obtain a transfection mixture, and the transfection mixture was completely added to 50 mL of the ExpiCHO cell solution within 5 minutes; (5) the cells were cultured under conditions of 37°C and 5% CO₂ for 1 day, 8 mL of Feed and 300µL of Enhancer (purchased from Thermo Fisher) were added into the solution, and cultured under conditions of 32°C and 5% CO₂ for 9 days and the culture supernatant was harvested, with 8 mL of Feed added on Day 5; and (6) affinity purification was performed on the culture supernatant by utilizing a Protein A purification column (purchased from Nano-micro) to obtain the target antibody.

In this example, a total of 11 CD3 and MICA bispecific antibodies were prepared to evaluate the various properties of the bispecific antibodies of the present disclosure. For details on the configuration and amino acid sequence of each antibody, reference can be made to FIG. 1, Table 1, and Table 2.

**[Table 2] Structure and Corresponding Amino Acid Sequence of Bispecific Antibody of Example 1**

| Antibody Name | Antibody Configuration | First Antigen-Binding Region | Second Antigen-Binding Region |
|---|---|---|---|
| Cross3×h5A1002 1:1 | A | SEQ ID NO: 16 | SEQ ID NO: 14 SEQ ID NO: 15 |
| Cross3×h5A1002 2:1 | B | SEQ ID NO: 13 | SEQ ID NO: 14 |
| | | SEQ ID NO: 15 | SEQ ID NO: 15 |
| UCHT1×h5A1002 1:1 | A | SEQ ID NO: 17 | SEQ ID NO: 14 |
| | | | SEQ ID NO: 15 |
| UCHT1×h5A1002 2:1 | B | SEQ ID NO: 18 | SEQ ID NO: 14 |
| | | SEQ ID NO: 15 | SEQ ID NO: 15 |
| SP34×h5A1002 2:1 | B | SEQ ID NO: 19 | SEQ ID NO: 14 |
| | | SEQ ID NO: 15 | SEQ ID NO: 15 |
| Cross3×1D5 1:1 | A | SEQ ID NO: 16 | SEQ ID NO: 20 |
| | | | SEQ ID NO: 21 |
| Cross3×1D5 2:1 | B | SEQ ID NO: 22 | SEQ ID NO: 20 |
| | | SEQ ID NO: 21 | SEQ ID NO: 21 |
| Cross3×13A9 1:1 | A | SEQ ID NO: 16 | SEQ ID NO: 23 |
| | | | SEQ ID NO: 24 |
| Cross3×13A9 2:1 | B | SEQ ID NO: 25 | SEQ ID NO: 23 |
| | | SEQ ID NO: 24 | SEQ ID NO: 24 |
| Cross3×619 1:1 | A | SEQ ID NO: 16 | SEQ ID NO: 26 |
| | | | SEQ ID NO: 27 |
| Cross3×619 2:1 | B | SEQ ID NO: 28 | SEQ ID NO: 26 |
| | | SEQ ID NO: 27 | SEQ ID NO: 27 |

| | | | |
|---|---|---|---|
| Note: The configurations of the antibodies A and B are shown in FIG. 1. Specifically, for Cross3×h5A1002 1:1, the amino acid sequence of the CD3 single-chain antibody is as set forth in SEQ ID NO: 16, the amino acid sequence of the MICA antibody heavy chain is as set forth in SEQ ID NO: 14, and the amino acid sequence of the MICA antibody light chain is as set forth in SEQ ID NO: 15; for Cross3×h5A1002 2:1, the amino acid sequence of the MICA antibody heavy chain-CD3 single chain antibody is as set forth in SEQ ID NO: 13, the amino acid sequence of the MICA antibody heavy chain is as set forth in SEQ ID NO: 14, and the amino acid sequence of the MICA antibody light chain is as set forth in SEQ ID NO: 15; for UCHT1×h5A1002 1:1, the amino acid sequence of the CD3 single-chain antibody is as set forth in SEQ ID NO: 17, the amino acid sequence of the MICA antibody heavy chain is as set forth in SEQ ID NO: 14, and the amino acid sequence of the MICA antibody light chain is as set forth in SEQ ID NO: 15; for UCHT1×h5A1002 2:1, the amino acid sequence of the MICA antibody heavy chain-CD3 single chain antibody is as set forth in SEQ ID NO: 18, the amino acid sequence of the MICA antibody heavy chain is as set forth in SEQ ID NO: 14, and the amino acid sequence of the MICA antibody light chain is as set forth in SEQ ID NO: 15; for SP34×h5A1002 2:1, the amino acid sequence of the MICA antibody heavy chain-CD3 single chain antibody is as set forth in SEQ ID NO: 19, the amino acid sequence of the MICA antibody heavy chain is as set forth in SEQ ID NO:14, and the amino acid sequence of the MICA antibody light chain is as set forth in SEQ ID NO: 15; for Cross3×1D5 1:1, the amino acid sequence of the CD3 single-chain antibody is as set forth in SEQ ID NO: 16, the amino acid sequence of the MICA antibody heavy chain is as set forth in SEQ ID NO: 20, and the amino acid sequence of the MICA antibody light chain is as set forth in SEQ ID NO: 21; for Cross3×1D5 2:1, the amino acid sequence of theMICA antibody heavy chain-CD3 single chain antibody is as set forth in SEQ ID NO: 22, the amino acid sequence of the MICA antibody heavy chain is as set forth in SEQ ID NO: 20, and the amino acid sequence of the MICA antibody light chain is as set forth in SEQ ID NO: 21; for Cross3×13A9 1:1, the amino acid sequence of the CD3 single-chain antibody is as set forth in SEQ ID NO: 16, the amino acid sequence of the MICA antibody heavy chain is as set forth in SEQ ID NO: 23, and the amino acid sequence of the MICA antibody light chain is as set forth in SEQ ID NO: 24; for Cross3×13A9 2:1, the amino acid sequence of the MICA antibody heavy chain-CD3 single chain antibody is as set forth in SEQ ID NO: 25, the amino acid sequence of the MICA antibody heavy chain is as set forth in SEQ ID NO: 23, and the amino acid sequence of the MICA antibody light chain is as set forth in SEQ ID NO: 24; for Cross3×619 1:1, the amino acid sequence of the CD3 single-chain antibody is as set forth in SEQ ID NO: 16, the amino acid sequence of the MICA antibody heavy chain is as set forth in SEQ ID NO: 26, and the amino acid sequence of the MICA antibody light chain is as set forth in SEQ ID NO: 27; and for Cross3×619 2:1, the amino acid sequence of the MICA antibody heavy chain-CD3 single chain antibody is as set forth in SEQ ID NO: 28, the amino acid sequence of the MICA antibody heavy chain is as set forth in SEQ ID NO: 26, and the amino acid sequence of the MICA antibody light chain is as set forth in SEQ ID NO: 27. | | | |

### Example 2: Antibody ELISA Binding Assay of the Present Disclosure

ELISA assay was used to detect the binding properties of the bispecific antibody. The antigen protein was coated into a 96-well plate, and the intensity of the signal after the addition of the antibody was used to determine the binding properties of the bispecific antibody and the antigen protein.
(1) MICA-His protein (purchased from Acro) was diluted to a concentration of 2 µg/ml with PBS buffer and aliquoted to a 96-well plate at a volume of 100 µl/well. The plate was placed at 4°C overnight. The PBS buffer in the 96-well plate was aspirated. The plate was washed 6 times with PBST (PBS containing 0.1% Tween 20, pH 7.2) buffer, and then 200 µl/well of PBS/10% BSA was added. The plate was incubated at 37°C for 2 hours for blocking. The blocking solution was removed, and the plate was washed 6 times with PBST, and Cross3×h5A1002 1:1, Cross3×h5A1002 2:1, and UCHT1×h5A1002 2:1 bispecific antibodies to be tested, and control hIgG1LALA (purchased from Biointron), which were serially diluted (at a maximum concentration of 20 µg/ml, 8 gradients, 5-fold dilution) with PBST/0.05% BSA, were added at a volume of 100 µl/well. The plate was incubated at 37°C for 1 hour. The reaction system was removed, and the plate was washed 6 times with PBST, and then HRP (horseradish peroxidase)-labeled anti-human IgG secondary antibody (purchased from Jacksonlab) diluted with PBST/0.05% BSA was added at 100 µl/well. The plate was incubated at 37°C for 1 hour. After the plate was washed 6 times with PBST, 80 µl/well of TMB (tetramethylbenzidine) was added, and the plate was incubated at room temperature for 3 minutes. 80 µl/well of 4M sulfuric acid was added to terminate the reaction. The absorbance was read at 450 nm using an enzyme reader.
   The results were shown in FIG. 2. The Cross3×h5A1002 1:1, Cross3×h5A1002 2:1, and UCHT1×h5A1002 2:1 antibodies can all bind to the MICA protein, with comparable binding capabilities.
(2) CD3E&D protein (purchased from Acro) was diluted to a concentration of 2 µg/ml with PBS buffer and aliquoted to a 96-well plate at a volume of 100 µl/well. The plate was placed at 4°C overnight. The PBS buffer in the 96-well plate was aspirated. The plate was washed 6 times with PBST (PBS containing 0.1% Tween 20, pH 7.2) buffer, and then 200 µl/well of PBS/10% BSA was added. The plate was incubated at 37°C for 2 hours for blocking. The blocking solution was removed, and the plate was washed 6 times with PBST, and Cross3×h5A1002 1:1, Cross3×h5A1002 2:1, and UCHT1×h5A1002 2:1 bispecific antibodies to be tested, and control hIgG1LALA (purchased from Biointron), which were serially diluted (at a maximum concentration of 20 µg/ml, 8 gradients, 5-fold dilution) with PBST/0.05% BSA, were added at a volume of 100 µl/well. The plate was incubated at 37°C for 1 hour. The reaction system was removed, and the plate was washed 6 times with PBST, and then HRP (horseradish peroxidase)-labeled anti-human IgG secondary antibody (purchased from Southern biotech) diluted with PBST/0.05% BSA were added at 100 µl/well. The plate was incubated at 37°C for 1 hour. After the plate was washed 6 times with PBST, 80 µl/well of TMB (tetramethylbenzidine) was added, and the plate was incubated at room temperature for 3 minutes. 80 µl/well of 4M sulfuric acid was added to terminate the reaction. The absorbance was read at 450 nm using an enzyme reader.

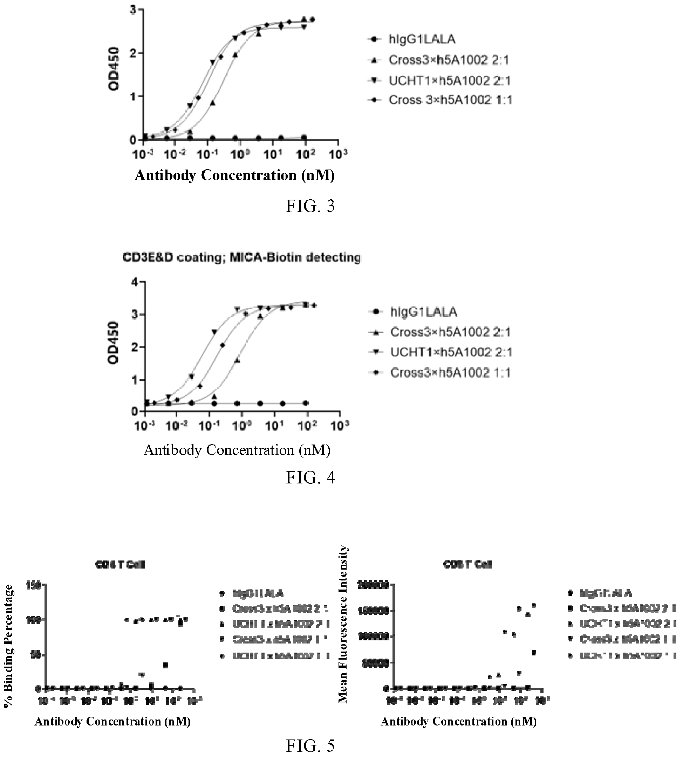

The results were shown in FIG. 3. The Cross3×h5A1002 1:1, Cross3×h5A1002 2:1, and UCHT1×h5A1002 2:1 antibodies can all bind to the CD3E&D protein, the capability of the Cross3×h5A1002 2:1 antibody to bind to CD3E&D was weaker than that of the Cross3×h5A1002 1:1 antibody; and the Cross3×h5A1002 2:1 antibody had the weakest activity in binding to CD3.

The above experimental results indicated that after adjusting the structure of the bispecific antibody (FIG. 1, configuration B), the antigen-binding fragment of the anti-CD3 antibody was successfully masked in configuration, and its capability to bind CD3 was weakened.

### Example 3: Antibody ELISA Bridging Assay

ELISA assay was used to detect the bridging binding properties of the bispecific antibody. CD3E&D antigen protein was coated into a 96-well plate. After the antibody was added, the biotin-labeled MICA protein was used for detection. The intensity of the signal was used to determine the binding properties of the bispecific antibody bridging the CD3E&D and MICA proteins.

MICA-His protein (purchased from Acro) was diluted to a concentration of 2 µg/ml with PBS buffer and aliquoted to a 96-well plate at a volume of 100 µl/well. The plate was placed at 4°C overnight. The PBS buffer in the 96-well plate was aspirated. The plate was washed 6 times with PBST (PBS containing 0.1% Tween 20, pH 7.2) buffer, and then PBS/10% BSA was added at 200 µl/well. The plate was incubated at 37°C for 2 hours for blocking. The blocking solution was removed, and the plate was washed 6 times with PBST, and Cross3×h5A1002 1:1, Cross3×h5A1002 2:1, and UCHT1×h5A1002 2:1 bispecific antibodies to be tested, and control hIgG1LALA (purchased from Biointron), which were serially diluted (at a maximum concentration of 20 µg/ml, 8 gradients, 5-fold dilution) with PBST/0.05% BSA, were added at a volume of 100 µl/well. The plate was incubated at 37°C for 1 hour. The reaction system was removed, and the plate was washed 6 times with PBST. MICA*002α3-Fc-Biotin diluted to an appropriate concentration was added into the plate, and the plate was incubated at 37°C for 1 hour. The reaction system was removed, and the plate was washed 6 times with PBST, and then HRP (horseradish peroxidase)-labeled Streptavidin secondary antibody (purchased from Southern Biotech) diluted with PBST/0.05% BSA was added at 100 µl/well. The plate was incubated at 37°C for 1 hour. After the plate was washed 6 times with PBST, TMB (tetramethylbenzidine) was added at 80 µl/well, and the plate was incubated at room temperature for 3 minutes. 80 µl/well of 4M sulfuric acid was added to terminate the reaction. The absorbance was read at 450 nm using an enzyme reader.

The results were shown in FIG. 4, the Cross3×h5A1002 1:1, Cross3×h5A1002 2:1, and UCHT1×h5A1002 2:1 antibodies can bridge the CD3E&D and MICA proteins; the bridging capability of the Cross3×h5A1002 2:1 antibody was weaker than that of the Cross3×h5A1002 1:1 antibody; and the Cross3×h5A1002 2:1 antibody had the weakest bridging capability.

Based on the results in FIG. 2 and FIG. 3, the weak bridging capability of the Cross3×h5A1002 2:1 antibody was attributed to its low affinity to CD3.

### Example 4: Antibody Flow Cytometry Binding Assay

Flow cytometry assay was used to detect the binding properties of the bispecific antibody. The bispecific antibody was added to cells, and the intensity of the signal after the addition of the antibody was used to determine the binding properties of the antibody and the cell.
(1) PBMCs were diluted to 2×10⁶/ml with PBS and aliquoted into a 1.5 ml Eppendorf (EP) tube at a volume of 100 µl/tube. 10 µl/tube of goat serum was added to each tube, and incubated at 4°C for 30 minutes for blocking. Cross3×h5A1002 1:1, Cross3×h5A1002 2:1, UCHT1×h5A1002 1:1, and UCHT1×h5A1002 2:1 bispecific antibodies, and control hIgG1LALA (purchased from Biointron) which were serially diluted were added, and the cells were incubated at 4°C for 30 minutes. 1 ml of PBS was added to the EP tube, and the EP tube was centrifuged at 3,500 rpm for 5 minutes at 4°C. The supernatant was discarded, and the cells were washed again with PBS. After centrifugation, the supernatant was discarded, and the cells were resuspended with 100 µl/tube of PBS. 1 µl/tube of Alexa-647-labeled goat anti-human IgG secondary antibody (purchased from Jackson Lab) and 0.5 µl/tube of PerCP-Cy5.5-labeled goat anti-human CD8 antibody were added, and incubated at 4°C in the dark for 30 minutes. The cells were washed twice with PBS, and the supernatant was discarded after centrifugation. The cells were resuspended with 200 µl/tube of PBS and detected using a flow cytometry.
   The results were shown in FIG. 5, the Cross3×h5A1002 1:1, Cross3×h5A1002 2:1, UCHT1×h5A1002 1:1, and UCHT1×h5A1002 2:1 antibodies can bind to CD8 T cells, and the bispecific antibody with a 2:1 configuration (configuration B) had a weaker T cell binding capability than the bispecific antibody with 1:1 configuration (configuration A). Among them, the bispecific antibody Cross3×h5A1002 2:1 had the weakest T cell binding activity.
(2) HCT-15 tumor cells were diluted to 2×10⁶/mL with PBS and aliquoted into a 1.5 ml EP tube at a volume of 100 µl/tube. 10 µl/tube of goat serum was added to each tube, and incubated at 4°C for 30 minutes for blocking. Cross3×h5A1002 1:1, Cross3×h5A1002 2:1, and UCHT1×h5A1002 2:1 bispecific antibodies, and control hIgG1LALA (purchased from Biointron) which were serially diluted were added, and the cells were incubated at 4°C for 30 min. 1 ml of PBS was added to the EP tube, and the EP tube was centrifuged at 3,500 rpm for 5 minutes at 4°C. The supernatant was discarded, and the cells were washed again with PBS. After centrifugation, the supernatant was discarded, and the cells were resuspended with 100 µl/tube of PBS. 1 µl/tube of Alexa-647-labeled goat anti-human IgG secondary antibody (purchased from Jackson Lab) was added, and incubated at 4°C in the dark for 30 minutes. The cells were washed twice with PBS, and the supernatant was discarded after centrifugation. The cells were resuspended with 200 µl/tube of PBS and detected using a flow cytometry.

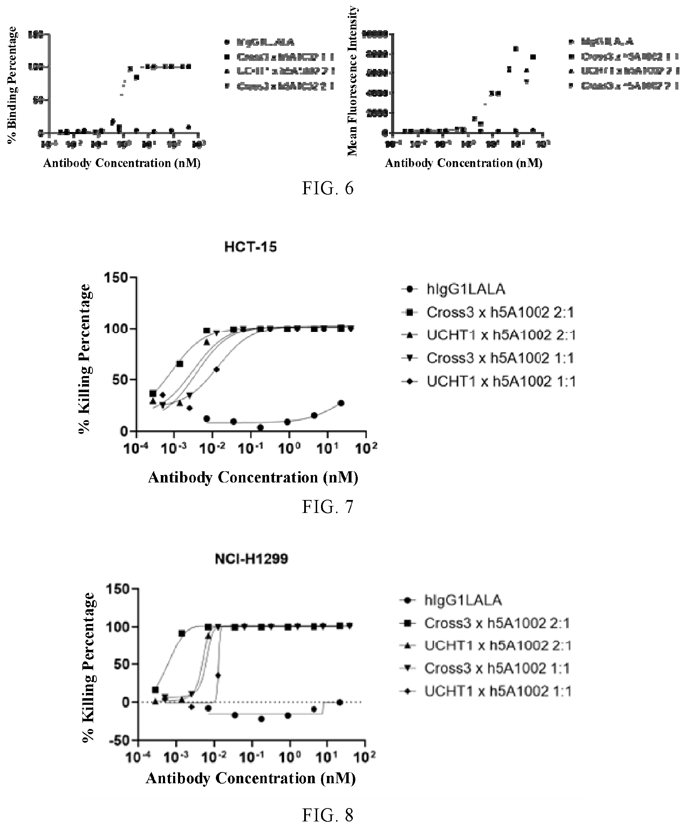

The results were shown in FIG. 6, (1) the Cross3×h5A1002 1:1, Cross3×h5A1002 2:1, and UCHT1×h5A1002 2:1 antibodies can bind to tumor cells; and (2) unexpectedly to the inventors, the bispecific antibody with 2:1 a configuration (configuration B) had a lower EC₅₀ for binding to HCT-15 tumor cells than the bispecific antibody with a 1:1 configuration (configuration A), indicating a stronger binding ability to the tumor cells. Among them, the antibodies Cross3×h5A1002 2:1 and UCHT1×h5A1002 2:1 had comparable activity in binding to tumors.

### Example 5: Assay on Bispecific Antibody Promoting PBMC-mediated Killing of Tumor Cells

Capability of the bispecific antibody to promote PBMC-mediated killing of HCT-15 colorectal cancer cells, NCI-H1299 lung cancer cells, PANC-1 pancreatic cancer cells, A-498 renal cancer cells, and PC-3 prostate cancer cells was detected.
(a) Complete RPMI-1640 medium was added into a 16-well RTCA plate at a volume of 50 µL/well, and calibration was conducted on a machine.
(b) The tumor cells were diluted to a density of 2×10⁵/mL with the complete RPMI-1640 medium and added individually into the RTCA plate obtained in the step (1) at a volume of 50 µL/well. Then, a cell coefficient was detected for 24 hours under conditions of 37°C and 5% CO₂ using an instrument xCELLigence RTCA MP.
(c) Cross3×h5A1002 1:1, Cross3×h5A1002 2:1, UCHT1×h5A1002 1:1, and UCHT1×h5A1002 2:1 bispecific antibodies, and control hIgG1LALA (purchased from Biointron), which were serially diluted (at a maximum concentration 0.2 µg/mL, 8 gradients, 5-fold dilution) using the complete RPMI-1640 medium, were added into the RTCA plate obtained in the step (2), with an addition volume of 20 µl/well.

(4) PBMCs (purchased from Shanghai Saily Biotechnology Co., Ltd.) were diluted to a density of 1.25×10⁶/mL with the complete RPMI-1640 medium, and added into the RTCA plate obtained in the step (3) at a volume of 80 µL/well.

(5) The reaction system obtained in the step (4) was placed under conditions of 37°C and 5% CO₂, and the cell coefficient was detected for 24 hours using an instrument xCELLigence RTCA MP.

The results were shown in FIG. 7, (1) the Cross3×h5A1002 1:1, Cross3×h5A1002 2:1, UCHT1×h5A1002 1:1, and UCHT1×h5A1002 2:1 antibodies promoted the PBMC-mediated killing of the HCT-15 colorectal cancer cells; and (2) unexpectedly to the inventors, the bispecific antibody with 2:1 configuration (configuration B) had a stronger pro-killing capability than the bispecific antibody with 1:1 configuration (configuration A), and the Cross3×h5A1002 2:1 antibody had the highest pro-killing activity.

The results were shown in FIG. 8, (1) the Cross3×h5A1002 1:1, Cross3×h5A1002 2:1, UCHT1×h5A1002 1:1, and UCHT1×h5A1002 2:1 antibodies promoted PBMC-mediated killing of the NCI-H1299 lung cancer cells; and (2) unexpectedly to the inventors, the bispecific antibody with a 2:1 configuration (configuration B) had a stronger pro-killing capability than the bispecific antibody with a 1:1 configuration (configuration A), and the Cross3×h5A1002 2:1 antibody had the highest pro-killing activity.

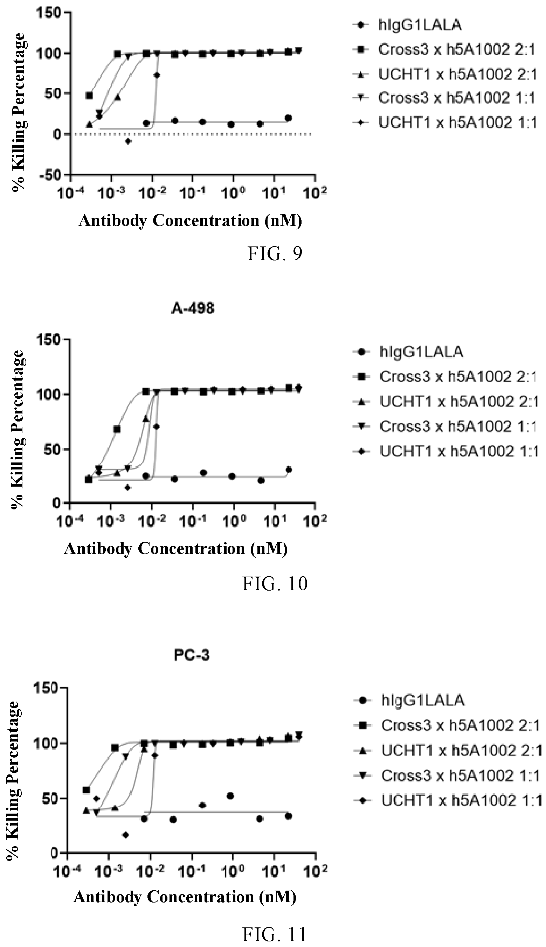

The results were shown in FIG. 9, (1) the Cross3×h5A1002 1:1, Cross3×h5A1002 2:1, UCHT1×h5A1002 1:1, and UCHT1×h5A1002 2:1 antibodies promoted PBMC-mediated killing of the PANC-1 pancreatic cancer cells; and (2) unexpectedly to the inventors, the bispecific antibody with the 2:1 configuration (configuration B) had a stronger pro-killing capability than thebispecific antibody with the 1:1 configuration (configuration A), and the Cross3×h5A1002 2:1 antibody had the highest pro-killing activity.

The results were shown in FIG. 10, (1) the Cross3×h5A1002 1:1, Cross3×h5A1002 2:1, UCHT1×h5A1002 1:1, and UCHT1×h5A1002 2:1 antibodies promoted PBMC-mediated killing of the A498 renal cancer cells. The bispecific antibody with the 2:1 configuration (configuration B) had a stronger pro-killing capability than the bispecific antibody with the 1:1 configuration (configuration A), and the Cross3×h5A1002 2:1 antibody had the highest pro-killing activity.

The results are shown in FIG. 11, (1) the Cross3×h5A1002 1:1, Cross3×h5A1002 2:1, UCHT1×h5A1002 1:1, and UCHT1×h5A1002 2:1 antibodies promoted PBMC-mediated killing of PC-3 prostate cancer cells; and (2) unexpectedly to the inventors, the bispecific antibody with the 2:1 configuration (configuration B) had a stronger pro-killing capability than the bispecific antibody with the 1:1 configuration (configuration A), and the Cross3×h5A1002 2:1 antibody had the highest pro-killing activity.

The above results showed that the capability of the bispecific antibody with the 2:1 configuration (configuration B) of the present disclosure to promote the effect of PBMCs for killing the HCT-15 rectal cancer cells, NCI-H1299 lung cancer cells, PANC-1 pancreatic cancer cells, A498 renal cancer cells, and PC-3 prostate cancer cells was significantly enhanced. When the CD3 antigen-binding region was the antigen-binding fragment of the Cross3 antibody, the bispecific antibody with the 2:1 configuration (configuration B) of the present disclosure had the strongest activity of promoting the PBMC-mediated killing of the tumor cells.

### Example 6: Assay on Bispecific Antibody Promoting Cytokine Secretion by PBMCs

The bispecific antibodies were added to a co-incubation system of PBMCs and tumor cells. After 48 hours of culture, the culture supernatant was collected, and the level of cytokines in the supernatant was detected to determine the properties of the bispecific antibody in inducing cytokine release.
(1) The tumor cells were diluted to 1×10⁵/mL using a complete RPMI 1640 medium and added to a 96-well plate. The plate was incubated in an incubator with 5% CO₂ at 37°C for 24 hours.
(2) The Cross3×h5A1002 1:1 and Cross3×h5A1002 2:1 bispecific antibodies, and control hIgG1LALA (purchased from Biointron) were serially diluted using complete RPMI 1640 medium, and added into the 96-well plate at a 20 µL/well.
(3) PBMCs (purchased from Shanghai Saily Biotechnology Co., Ltd.) were diluted to 1.25×10⁶/mL using a complete RPMI 1640 medium and added to the 96-well plate at 80 µL/well.
(4) The 96-well plate was incubated in an incubator with 5% CO₂ at 37°C for 48 hours.
(5) The cells were centrifuged at 300 g for 10 minutes at room temperature, and the cell culture supernatant was collected.
(6) The level of the cytokines in the supernatant was detected using a CBA kit (purchased from BD).

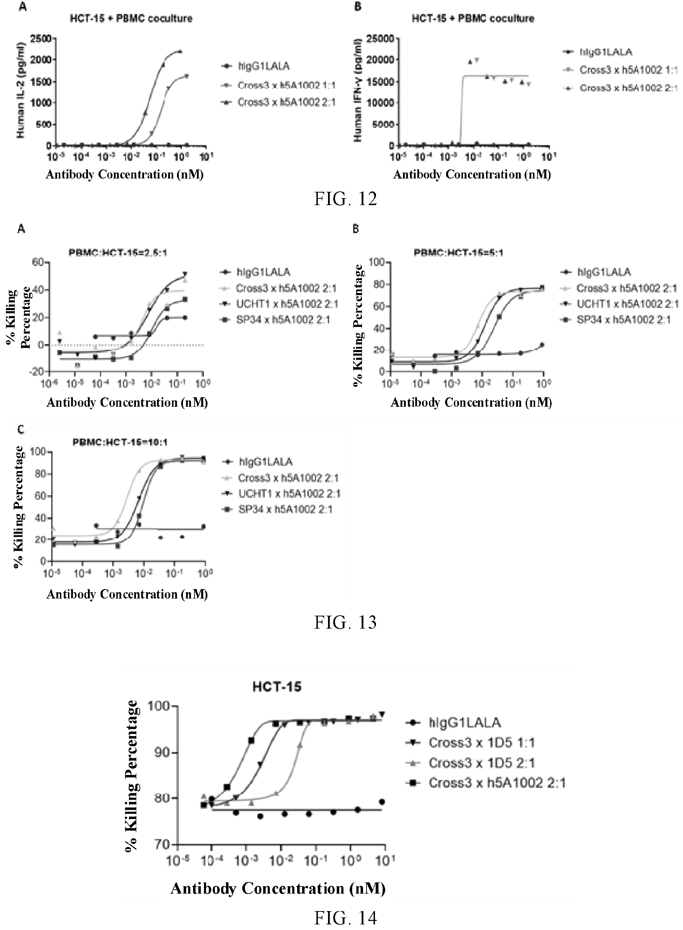

The results were shown in FIG. 12, (1) the Cross3×h5A1002 1:1 and Cross3×h5A1002 2:1 antibodies promoted the secretion of pro-inflammatory cytokines IL-2 and IFN-γ by PBMCs; and (2) the Cross3×h5A1002 2:1 antibody had a stronger capability to promote the secretion of the pro-inflammatory cytokines by PBMCs.

The above results showed that the bispecific antibody with the 2:1 configuration (configuration B) of the present disclosure had an enhanced capability to promote the secretion of the pro-inflammatory cytokines by PBMCs. Considering that thebispecific antibody with 2:1 configuration (configuration B) had weak binding to T cells but strong binding to the tumor cells, more drugs can be distributed to the tumor sites after administration, thus enhancing anti-cancer efficacy.

### Example 7: Assay on the Bispecific Antibody Promoting PBMC-mediated Killing of Tumor Cells at Various Effector-to-target Ratios

Capability of the bispecific antibody of the present disclosure promoting PBMC-mediated killing of HCT-15 colorectal cancer cells at different effector-to-target ratios (PBMC: tumor) was detected.
(1) Complete RPMI-1640 medium was added into a 16-well RTCA plate at a volume of 50 µL/well, and calibration was conducted on a machine.
(2) Tumor cells were diluted to a density of 2×10⁵/mL with the complete RPMI-1640 medium. The cells were added into the RTCA plate obtained in the step (1) at a volume of 50 µL/well. Then, a cell coefficient was detected for 24 hours under conditions of 37°C and 5% CO₂ using an instrument xCELLigence RTCA MP.
(3) Cross3×h5A1002 2:1, UCHT1×h5A1002 2:1, SP34×h5A1002 2:1 bispecific antibodies, and control hIgG1LALA (purchased from Biointron), which were serially diluted (at a maximum concentration 0.2 µg/mL, 8 gradients, 5-fold dilution) using the complete RPMI-1640 medium, were added into the RTCA plate obtained in the step (2), with an addition volume of 20 µL/well.
(4) PBMCs (purchased from Shanghai Saily Biotechnology Co., Ltd.) were diluted to densities of 1.25×10⁶ cells/mL (at an effector-to-target ratio of 10:1), 0.625×10⁶ cells/mL (at an effector-to-target ratio of 5:1), and 0.3125×10⁶ cells/mL (at an effector-to-target ratio of 2.5:1) with the complete RPMI-1640 medium, and added into the RTCA plate obtained in the step (3) at a volume of 80 µL/well.
(5) The reaction system obtained in the step (4) was placed under conditions of 37°C and 5% CO₂, and the cell coefficient was detected for 24 hours using an instrument xCELLigence RTCA MP.

The results were shown in FIG. 13. The Cross3×h5A1002 2:1, UCHT1×h5A1002 2:1, and SP34×h5A1002 2:1 antibodies all promoted the PBMC-mediated killing of the HCT-15 colorectal cancer cells at different effector-to-target ratios, and the Cross3×h5A1002 2:1 antibody exhibited the highest pro-killing activity.

### Example 8: Assay of Other MICA Bispecific Antibody in Promoting PBMC-mediated Killing of Tumor Cells

Capability of the bispecific antibody with the 2:1 configuration (configuration B) of the present disclosure formed based on the other MICA antibodies in promoting PBMC-mediated killing of HCT-15 colorectal cancer cells was detected to evaluate whether the capability of the bispecific antibody with the 2:1 configuration (configuration B) of the present disclosure in promoting PBMC-mediated killing of the tumor cells was generally superior to that of the bispecific antibody with the 1:1 configuration (configuration A). The other MICA antibodies were selected from 1D5 (Genentech monoclonal antibody, US20200055939A1), 13A9 (Genentech monoclonal antibody, US20200055939A1), and 619 (Cullinan monoclonal antibody, US20210253711A1).
1. The antibodies with the 2:1 configuration (configuration B) were expressed using the same method as described in Example 1.
2. Assessment of capability in promoting PBMC-mediated killing of tumor cells: Reference can be made to Example 7 in accordance with the following steps.

(1) Complete RPMI-1640 medium was added into a 16-well RTCA plate at a volume of 50 µL/well, and calibration was conducted on a machine.
(2) The tumor cells were diluted to a density of 2× 10⁵/mL with the complete RPMI-1640 medium and added to the RTCA plate obtained in the step (1) at a volume of 50 µL/well. Then, the cell coefficient was detected for 24 hours at under conditions of 37°C and 5% CO₂ using an instrument xCELLigence RTCA MP.
(3) Cross3×h5A1002 2:1, Cross3×1D5 1:1, Cross3×1D5 2:1, Cross3×13A9 1:1, Cross3×13A9 2:1, Cross3×619 1:1, and Cross3×619 2:1 bispecific antibodies, and control hIgG1LALA (purchased from Biointron), which were serially diluted (at a maximum concentration 0.2 µg/mL, 8 gradients, 5-fold dilution) using the complete RPMI-1640 medium, were added into the RTCA plate obtained in the step (2), with an addition volume of 20 µL/well.
(4) PBMCs (purchased from Shanghai Saily Biotechnology Co., Ltd.) were diluted to 1.25×10⁶ cells/mL with the complete RPMI-1640 medium and added into the RTCA plate obtained in the step (3) at a volume of 80 µL/well.
(5) The reaction system obtained in the step (4) was placed under conditions of 37°C and 5% CO₂, and the cell coefficient was detected for 24 hours using an instrument xCELLigence RTCA MP.

The results were shown in FIG. 14, (1) the 2:1 configuration bispecific antibody based on 1D5 had a weaker pro-killing capability than the 1:1 configuration bispecific antibody; and (2) the Cross3×h5A1002 2:1 antibody had higher pro-killing activity than the bispecific antibody based on 1D5.

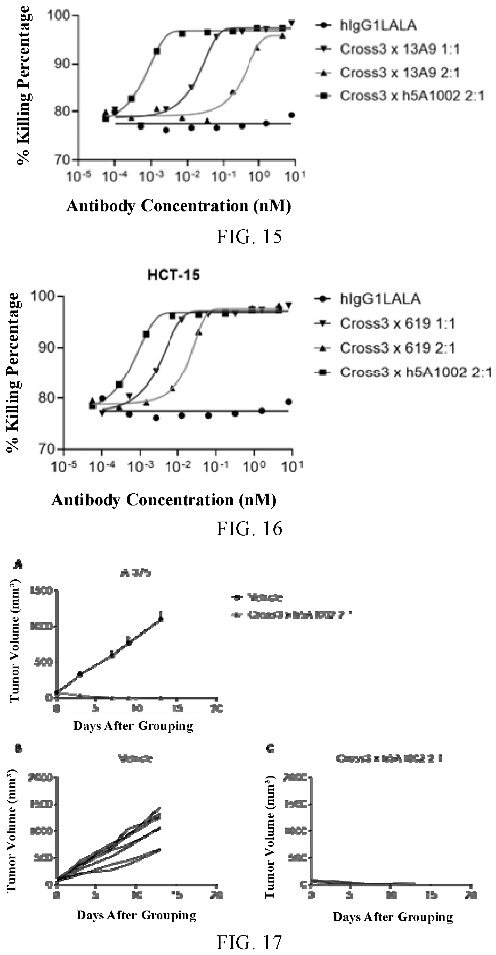

The results were shown in FIG. 15, (1) the 2:1 configuration bispecific antibody based on 13A9 had a weaker pro-killing capability than the 1:1 configuration bispecific antibody; and (2) the Cross3×h5A1002 2:1 antibody has higher pro-killing activity than the bispecific antibody based on 13A9.

The results were shown in FIG. 16, (1) the 2:1 configuration bispecific antibody based on 619 had a weaker pro-killing capability than the 1:1 configuration bispecific antibody, and (2) the Cross3×h5A1002 2:1 antibody had higher pro-killing activity than the bispecific antibody based on 619.

The above results showed that only when the MICA antibody was selected from the h5A1002 antibody, the 2:1 configuration (configuration B) bispecific antibody of the present disclosure exhibited an enhanced capability in promoting PBMC-mediated killing of the tumor cells.

### Example 9: Anti-cancer Effect of CD3×MICA Antibody in mice

In vivo efficacy assay was used to detect the antitumor-promoting function of the Cross3×h5A1002 2:1 bispecific antibody of the present disclosure in immune-reconstituted mice.
(1) On day -14, human PBMCs (purchased from Shanghai Saily Biotechnology Co., Ltd.) were transfused into NSG mice (purchased from Shanghai Model Organisms Center, Inc.) via the tail vein at a injection dose of 5×10⁶ cells/mouse.
(2) On day 10, the NSG mice were subcutaneously implanted with 2×10⁶ A-375 human melanoma cells, 2×10⁶ NCI-H1299 human lung cancer cells, or 5×10⁶ PC3 human prostate cancer cells in the right ventral flank.
(3) On day 0, the mice were weighed, and tumor volumes were measured. Based on the weighing results and tumor volumes, the mice were divided into 6 groups.
(4) On days 0, 4, 7, and 11, the mice were injected via the tail vein with the Cross3×h5A1002 2:1 antibody (the amino acid sequence of the MICA antibody heavy chain-CD3 single chain antibody as set forth in SEQ ID NO: 13, the amino acid sequence of the MICA antibody heavy chain as set forth in SEQ ID NO: 14, and the amino acid sequence of the MICA antibody light chain as set forth in SEQ ID NO: 15) (0.1 mg/kg) and the solvent control PBS at a volume of 250 µL/mouse.
(4) After the injection of the above-mentioned antibodies, the tumor volumes were measured twice a week, and the mice were weighed.

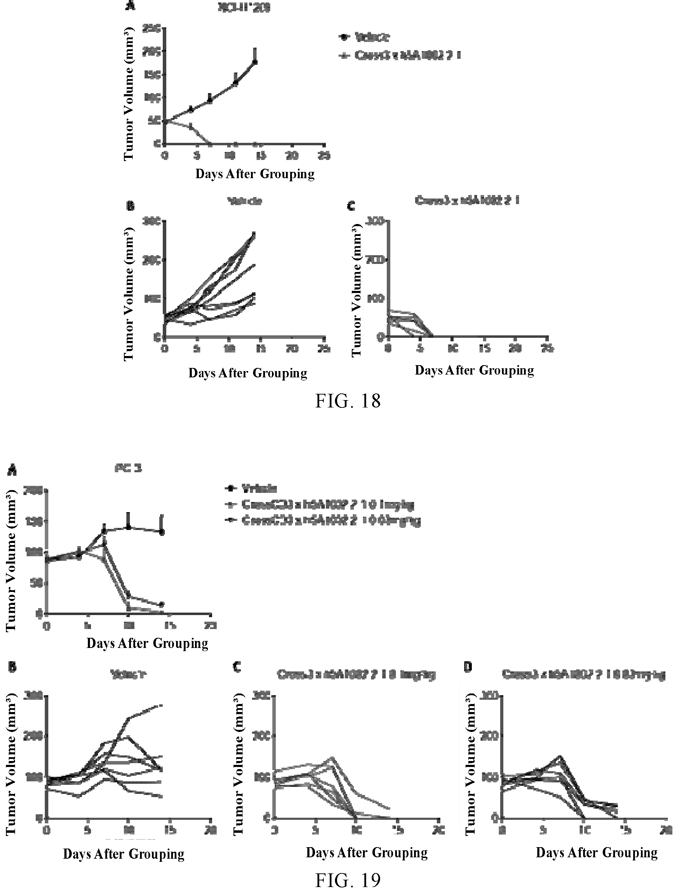

The results were shown in FIG. 17, FIG. 19, and FIG. 20, the Cross3×h5A1002 2:1 antibody of the present disclosure can effectively promote an anti-cancer effect of the immune-reconstituted mice, and the tumors can completely regress when the antibody was used for treating the tumor-bearing mice.

The above experimental results show that, the bispecific antibody of the present disclosure can bind to CD3 and MICA, thereby promoting T cell activation and secretion of cytokines, effectively promoting PBMC-mediated tumor cell killing, and exhibiting good anti-cancer activity. The bispecific antibody of the present disclosure can achieve binding to MICA with high-affinity and binding to CD3 with low affinity, thus exhibiting higher anti-cancer activity and better safety. In summary, the bispecific antibody of the present disclosure can promote anti-cancer effects of immune cells, exhibits good anti-cancer activity and higher safety, and has high clinical application value and drug development value.

The preferred embodiments of the present disclosure are described in detail above. However, the present disclosure is not limited thereto. Without departing from the scope of the technical concept of the present disclosure, various simple modifications can be made to the technical solutions of the present disclosure, including the combination of various technical features in any other suitable manner. These simple modifications and combinations should also be regarded as the disclosed content of the present disclosure and fall within the protection scope of the present disclosure.

In the description of the present specification, the descriptions of the reference terms such as "one embodiment", "some embodiments", "an example", "a specific example" and "some examples" mean that the specific features, structures, materials or characteristics described in combination with the embodiments or examples are included in at least one embodiment or example of the present disclosure. In the present specification, the schematic expressions of the above terms do not necessarily refer to the same embodiment or example. Moreover, the specific features, structures, materials or characteristics described herein may be combined in any one or more embodiments or examples in an appropriate mode. In addition, in absence of mutual contradiction, different embodiments or examples and features of the different embodiments or examples described in the present specification may be combined and integrated by those skilled in the art.

Although the embodiments of the present disclosure have been illustrated and described above, it can be understood that the above embodiments are illustrative and should not be construed as limiting the present disclosure. Those skilled in the art can make changes, modifications, substitutions, and variations to the above embodiments within the scope of the present disclosure.

## Claims

1. A bispecific antibody, comprising:
a first antigen-binding region, comprising an scFv fragment and a binding protein or fragment thereof of a first molecule;
a second antigen-binding region, comprising a binding protein or fragment thereof of a second molecule; and
an Fc portion, comprising a first Fc peptide fragment and a second Fc peptide fragment, wherein:
the binding protein or fragment thereof of the first molecule is linked to an N-terminus of the scFv fragment, and a C-terminus of the scFv fragment is linked to an N-terminus of the first Fc peptide fragment;
the binding protein or fragment thereof of the second molecule is linked to an N-terminus of the second Fc peptide fragment; and
the first molecule and the second molecule are identical, and the scFv fragment has CD3 binding activity.

2. The bispecific antibody according to claim 1, wherein the first molecule and the second molecule are each selected from MICA, PDL1, CD47, TIGIT, CD73, CD33, CEACAM1, CEACAM5, CEACAM6, STING, WNT, Beta catenin, B7H3, VISITA, CD19, BCMA, CD22, CD20, CD123, CD38, CEA, CD25, CD46, CD138, PSCA, PSMA, PSA, MUC1, MUC16, NY-ESO-1, GD2, WT1, Mesothelin, MAGE-A3, GPC3, PRAME, Globo H, AFP, Trop2, FOLR1, SP, Sca-1, CD133, or EPCAM.

3. The bispecific antibody according to claim 2, wherein both the first molecule and the second molecule are MICA.

4. The bispecific antibody according to claim 1, wherein the binding protein or fragment thereof is selected from at least one of an antibody or antigen-binding fragment thereof, a receptor, or a ligand.

5. The bispecific antibody according to claim 1, wherein the binding protein or fragment thereof of the first molecule and/or the binding protein or fragment thereof of the second molecule is independently selected from a Fab fragment or a scFab fragment.

6. The bispecific antibody according to claim 1, wherein:
the binding protein or fragment thereof of the first molecule is a first Fab fragment; and/or
the binding protein or fragment thereof of the second molecule is a second Fab fragment.

7. The bispecific antibody according to claim 6, wherein:
a C-terminus of a CH1 fragment of the first Fab fragment is linked to the N-terminus of the scFv fragment, and the C-terminus of the scFv fragment is linked to the N-terminus of the first Fc peptide fragment; and/or
a C-terminus of a CH1 fragment of the second Fab fragment is linked to the N-terminus of the second Fc peptide fragment.

8. The bispecific antibody according to claim 6, wherein both the first Fab fragment and the second Fab fragment have MICA binding activity.

9. The bispecific antibody according to claim 6, wherein both the first Fab fragment and the second Fab fragment comprise second heavy chain variable regions CDR1 to CDR3 and second light chain variable regions CDR1 to CDR3, wherein:
the second heavy chain variable region CDR1 has an amino acid sequence as set forth in SEQ ID NO: 7 or a conservative modification form thereof;
the second heavy chain variable region CDR2 has an amino acid sequence as set forth in SEQ ID NO: 8 or a conservative modification form thereof;
the second heavy chain variable region CDR3 has an amino acid sequence as set forth in SEQ ID NO: 9 or a conservative modification form thereof;
the second light chain variable region CDR1 has an amino acid sequence as set forth in SEQ ID NO: 10 or a conservative modification form thereof;
the second light chain variable region CDR2 has an amino acid sequence as set forth in SEQ ID NO: 11 or a conservative modification form thereof; and
the second light chain variable region CDR3 has an amino acid sequence as set forth in SEQ ID NO: 12 or a conservative modification form thereof.

10. The bispecific antibody according to claim 6, wherein the first Fab fragment and/or the second Fab fragment comprises a second heavy chain framework region and/or a second light chain framework region.

11. The bispecific antibody according to claim 10, wherein at least a portion of the second heavy chain framework region and/or the second light chain framework region is from at least one of a murine antibody, a humanized antibody, a primate-derived antibody, a bovine-derived antibody, an equine-derived antibody, a dairy bovine-derived antibody, a porcine-derived antibody, a sheep-derived antibody, a goat-derived antibody, a canine-derived antibody, a feline-derived antibody, a rabbit-derived antibody, a camel-derived antibody, a donkey-derived antibody, a deer-derived antibody, a mink-derived antibody, a chicken-derived antibody, a duck-derived antibody, a goose-derived antibody, a turkey-derived antibody, a gamecock-derived antibody, or a mutant thereof, and preferably, from at least one of a murine antibody, a humanized antibody, or a primate-derived antibody.

12. The bispecific antibody according to claim 6, wherein the first Fab fragment and the second Fab fragment each independently comprise:
a second heavy chain variable region having an amino acid sequence as set forth in SEQ ID NO: 29 or a conservative modification form thereof; and
a second light chain variable region having an amino acid sequence as set forth in SEQ ID NO: 30 or a conservative modification form thereof.

13. The bispecific antibody according to claim 6, wherein the first Fab fragment and/or the second Fab fragment further comprises a CH1 fragment and a CL fragment, wherein:
a C-terminus of a second heavy chain variable region is linked to an N-terminus of the CH1 fragment;
a C-terminus of a second light chain variable region is linked to an N-terminus of the CL fragment; and
the CH1 fragment is linked to the CL fragment via an interchain disulfide bond.

14. The bispecific antibody according to claim 6, wherein the first Fab fragment and the second Fab fragment each independently comprise:
a second heavy chain variable region and a CH1 fragment, having an amino acid sequence as set forth in SEQ ID NO: 31; and
a second light chain variable region and a CL fragment, having an amino acid sequence as set forth in SEQ ID NO: 32.

15. The bispecific antibody according to any one of claims 1 to 14, wherein the scFv fragment comprises first heavy chain variable regions CDR1 to CDR3 and first light chain variable regions CDR1 to CDR3, wherein:
the first heavy chain variable region CDR1 has an amino acid sequence as set forth in SEQ ID NO: 1;
the first heavy chain variable region CDR2 has an amino acid sequence as set forth in SEQ ID NO: 2;
the first heavy chain variable region CDR3 has an amino acid sequence as set forth in SEQ ID NO: 3;
the first light chain variable region CDR1 has an amino acid sequence as set forth in SEQ ID NO: 4;
the first light chain variable region CDR2 has an amino acid sequence as set forth in SEQ ID NO: 5; and
the first light chain variable region CDR3 has an amino acid sequence as set forth in SEQ ID NO: 6.

16. The bispecific antibody according to any one of claims 1 to 14, wherein the scFv fragment comprises a first heavy chain framework region and/or a first light chain framework region.

17. The bispecific antibody according to claim 16, wherein at least a portion of the first heavy chain framework region and/or the first light chain framework region is from at least one of a murine antibody, a humanized antibody, a primate-derived antibody, a bovine-derived antibody, an equine-derived antibody, a dairy bovine-derived antibody, a porcine-derived antibody, a sheep-derived antibody, a goat-derived antibody, a canine-derived antibody, a feline-derived antibody, a rabbit-derived antibody, a camel-derived antibody, a donkey-derived antibody, a deer-derived antibody, a mink-derived antibody, a chicken-derived antibody, a duck-derived antibody, a goose-derived antibody, a turkey-derived antibody, a gamecock-derived antibody, or a mutant thereof, and preferably, from at least one of a murine antibody, a humanized antibody, or a primate-derived antibody.

18. The bispecific antibody according to any one of claims 1 to 14, wherein the scFv fragment comprises:
a first heavy chain variable region having an amino acid sequence as set forth in SEQ ID NO: 33 or a conservative modification form thereof; and
a first light chain variable region having an amino acid sequence as set forth in SEQ ID NO: 34 or a conservative modification form thereof.

19. The bispecific antibody according to claim 18, wherein the scFv fragment further comprises a linker peptide 1, wherein:
an N-terminus of the linker peptide 1 is linked to a C-terminus of the first heavy chain variable region, and a C-terminus of the linker peptide 1 is linked to an N-terminus of the first light chain variable region; or
the N-terminus of the linker peptide 1 is linked to a C-terminus of the first light chain variable region, and the C-terminus of the linker peptide 1 is linked to an N-terminus of the first heavy chain variable region.

20. The bispecific antibody according to claim 19, wherein the linker peptide 1 has an amino acid sequence as set forth in (GGGGS)n, where n is an integer greater than or equal to 1, and preferably, 1, 2, 3, 4, 5, 6, 7, 8, 9, or 10.

21. The bispecific antibody according to claim 19, wherein the scFv fragment has an amino acid sequence as set forth in SEQ ID NO: 35.

22. The bispecific antibody according to claim 19, wherein the first antigen-binding region further comprises a linker peptide 2, wherein:
a C-terminus of a CH1 fragment of a first Fab fragment is linked to an N-terminus of the linker peptide 2; and
a C-terminus of the linker peptide 2 is linked to the N-terminus of the scFv fragment.

23. The bispecific antibody according to claim 22, wherein the linker peptide 2 has an amino acid sequence as set forth in (GGGGS)n, where n is an integer greater than or equal to 1, and preferably, 1, 2, 3, 4, 5, 6, 7, 8, 9 or 10.

24. The bispecific antibody according to claim 22, wherein the linker peptide 2 has an amino acid sequence as set forth in SEQ ID NO: 36 or SEQ ID NO: 37.

25. The bispecific antibody according to any one of claims 1 to 7, wherein the first Fc peptide fragment and the second Fc peptide fragment are linked via a knock-into-hole structure.

26. The bispecific antibody according to any one of claims 1 to 7, wherein both the first Fc peptide fragment and the second Fc peptide fragment are human Fc peptide fragments.

27. The bispecific antibody according to claim 26, wherein the human Fc peptide fragment is a human IgG1 Fc peptide fragment.

28. The bispecific antibody according to claim 26, wherein:
the first Fc peptide fragment has an amino acid sequence as set forth in SEQ ID NO: 38; and
the second Fc peptide fragment has an amino acid sequence as set forth in SEQ ID NO: 39 or SEQ ID NO: 40.

29. The bispecific antibody according to any one of claims 2 to 7, comprising:
a first polypeptide chain having an amino acid sequence as set forth in SEQ ID NO: 13, SEQ ID NO: 18, SEQ ID NO: 19, SEQ ID NO: 22, SEQ ID NO: 25, or SEQ ID NO: 28, or an amino acid sequence having at least 80% identity thereto;
a second polypeptide chain having an amino acid sequence as set forth in SEQ ID NO: 14, SEQ ID NO: 20, SEQ ID NO: 23, or SEQ ID NO: 26, or an amino acid sequence having at least 80% identity thereto;
a third polypeptide chain having an amino acid sequence as set forth in SEQ ID NO: 15, SEQ ID NO: 21, SEQ ID NO: 24, or SEQ ID NO: 27, or an amino acid sequence having at least 80% identity thereto; and
a fourth polypeptide chain having an amino acid sequence as set forth in SEQ ID NO: 15, SEQ ID NO: 21, SEQ ID NO: 24, or SEQ ID NO: 27, or an amino acid sequence having at least 80% identity thereto.

30. The bispecific antibody according to claim 29, comprising:
a first polypeptide chain having an amino acid sequence as set forth in SEQ ID NO: 13, a second polypeptide chain having an amino acid sequence as set forth in SEQ ID NO: 14, a third polypeptide chain having an amino acid sequence as set forth in SEQ ID NO: 15, and a fourth polypeptide chain having an amino acid sequence as set forth in SEQ ID NO: 15; or
a first polypeptide chain having an amino acid sequence as set forth in SEQ ID NO: 18, a second polypeptide chain having an amino acid sequence as set forth in SEQ ID NO: 14, a third polypeptide chain having an amino acid sequence as set forth in SEQ ID NO: 15, and a fourth polypeptide chain having an amino acid sequence as set forth in SEQ ID NO: 15; or
a first polypeptide chain having an amino acid sequence as set forth in SEQ ID NO: 19, a second polypeptide chain having an amino acid sequence as set forth in SEQ ID NO: 14, a third polypeptide chain having an amino acid sequence as set forth in SEQ ID NO: 15, and a fourth polypeptide chain having an amino acid sequence as set forth in SEQ ID NO: 15; or
a first polypeptide chain having an amino acid sequence as set forth in SEQ ID NO: 22, a second polypeptide chain having an amino acid sequence as set forth in SEQ ID NO: 20, a third polypeptide chain having an amino acid sequence as set forth in SEQ ID NO: 21, and a fourth polypeptide chain having an amino acid sequence as set forth in SEQ ID NO: 21; or
a first polypeptide chain having an amino acid sequence as set forth in SEQ ID NO: 25, a second polypeptide chain having an amino acid sequence as set forth in SEQ ID NO: 23, a third polypeptide chain having an amino acid sequence as set forth in SEQ ID NO: 24, and a fourth polypeptide chain having an amino acid sequence as set forth in SEQ ID NO: 24; or
a first polypeptide chain having an amino acid sequence as set forth in SEQ ID NO: 28, a second polypeptide chain having an amino acid sequence as set forth in SEQ ID NO: 26, a third polypeptide chain having an amino acid sequence as set forth in SEQ ID NO: 27, and a fourth polypeptide chain having an amino acid sequence as set forth in SEQ ID NO: 27.

31. A nucleic acid, encoding the bispecific antibody according to any one of claims 1 to 30.

32. A vector or transformant, comprising the nucleic acid according to claim 31.

33. The vector or transformant according to claim 32, wherein the vector is a eukaryotic vector or a prokaryotic vector.

34. The vector or transformant according to claim 32, wherein the vector comprises at least one selected from a plasmid vector, an adenoviral vector, a lentiviral vector, or an adeno-associated viral vector.

35. A cell, carrying the nucleic acid according to claim 31 or the vector or transformant according to claims 32 to 34, or expressing the multispecific antibody according to any one of claims 1 to 30.

36. The cell according to claim 35, wherein the cell is a prokaryotic cell, a eukaryotic cell, or a bacteriophage.

37. A pharmaceutical composition, comprising:
the multispecific antibody according to any one of claims 1 to 30;
the nucleic acid according to claim 31;
the vector or transformant according to any one of claims 32 to 34; or
the cell according to claim 35 or 36.

38. The pharmaceutical composition according to claim 37, further comprising a pharmaceutically acceptable adjuvant.

39. The pharmaceutical composition according to claim 38, wherein the adjuvant comprises one or more pharmaceutically acceptable excipients, diluents, stabilizers, or carriers.

40. The pharmaceutical composition according to claim 37, wherein the pharmaceutical composition is in the form of an injection.

41. A kit, comprising:
the multispecific antibody according to any one of claims 1 to 30;
the nucleic acid according to claim 31;
the vector or transformant according to claims 32 to 34; or
the cell according to claim 35 or 36.

42. Use of the multispecific antibody according to any one of claims 1 to 30, the nucleic acid according to claim 31, the vector or transformant according to claims 32 to 34, or the cell according to claim 35 or 36 in the manufacture of a kit for detecting CD3 and/or a tumor target.

43. Use of the multispecific antibody according to any one of claims 1 to 30, the nucleic acid according to claim 31, the vector or transformant according to claims 32 to 34, the cell according to claim 35 or 36, or the pharmaceutical composition according to claims 37 to 40 in the manufacture of a medicament for preventing and/or treating a CD3-mediated disease and/or a tumor target-mediated disease.

44. The use according to claim 43, wherein the CD3-mediated disease comprises an autoimmune disease.

45. The use according to claim 44, wherein the autoimmune disease comprises at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, bullous pemphigoid, primary biliary cirrhosis, multiple sclerosis, or acute idiopathic polyneuritis.

46. The use according to claim 43, wherein the tumor target is MICA.

47. The use according to claim 46, wherein the MICA-mediated disease comprises a cancer or transplant rejection, an autoimmune disease, or an infectious disease.

48. The use according to claim 47, wherein the cancer comprises at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head-and-neck cancer.

49. A method for preventing and/or treating a CD3-mediated disease and/or MICA-mediated disease, the method comprising:
administering to a subject a pharmaceutically acceptable amount of the bispecific antibody of claims 1 to 30, the nucleic acid according to claim 31, the vector or transformant according to claims 32 to 34, the cell of claim 35 or 36, or the pharmaceutical composition according to claims 37 to 40.

50. The method according to claim 49, wherein the CD3-mediated disease comprises an autoimmune disease.

51. The method according to claim 50, wherein the autoimmune disease comprises at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, bullous pemphigoid, primary biliary cirrhosis, multiple sclerosis, or acute idiopathic polyneuritis.

52. The method according to claim 51, wherein the tumor target is MICA.

53. The method according to claim 52, wherein the MICA-mediated disease comprises a cancer or transplant rejection, an autoimmune disease, or an infectious disease.

54. The method according to claim 53, wherein the cancer comprises at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, or head-and-neck cancer.
